# EUROPEAN PATENT APPLICATION

(11) **EP 4 711 370 A1**
(43) Date of publication of application: **18.03.2026**
(21) Application number: 24802928.2
(22) Date of filing: 06.05.2024
(51) Int. Cl.: C07D 491/22, A61K 31/4745, A61P 35/00

(54) **DEUTERATED CAMPTOTHECIN COMPOUND, AND PREPARATION THEREFOR AND USE THEREOF**

(30) Priority: 06.05.2023 CN 202310506357; 16.11.2023 CN 202311533730; 29.04.2024 CN 202410535444
(71) Applicant: Shanghai Qilu Pharmaceutical Research and Development Centre Ltd., Shanghai 201203 (CN)
(72) Inventor: LV, Wei, Shanghai 201203 (CN); JIN, Jiyu, Shanghai 201203 (CN); JIN, Chen, Shanghai 201203 (CN); YANG, Yang, Shanghai 201203 (CN); HUANG, Ying, Shanghai 201203 (CN); TAO, Weikang, Shanghai 201203 (CN)
(74) Representative: Dai, Simin
(86) International application number: PCT/CN2024/091220
(87) International publication number: WO 2024/230646

(57) **Abstract**

Disclosed are a deuterated camptothecin compound as represented by formula II and a pharmaceutically acceptable salt thereof, a preparation method therefor, a pharmaceutical composition containing the deuterated camptothecin compound or a pharmaceutically acceptable salt thereof, and the use thereof in the treatment of tumor-related diseases. Such deuterated camptothecin compound has excellent drug activity and pharmacokinetic properties, and has reduced in-vivo toxicity and improved in-vivo safety.

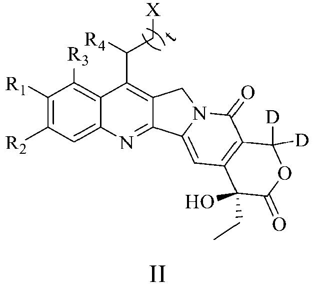

## Description

### CROSS-REFERENCE TO RELATED APPLICATIONS

The present application claims the benefits and priorities of Chinese Patent Application No. 202310506357.6 filed with the China National Intellectual Property Administration on May 6, 2023, Chinese Patent Application No. 202311533730.3 filed with the China National Intellectual Property Administration on November 16, 2023, and Chinese Patent Application No. 202410535444.9 filed with the China National Intellectual Property Administration on April 29, 2024, the entire contents of which are hereby incorporated by reference herein in their entirety.

### TECHNICAL FIELD

The present disclosure belongs to the field of medicinal chemistry, and specifically relates to a class of deuterated camptothecin compounds, pharmaceutically acceptable salts thereof, preparation methods therefor, pharmaceutical compositions containing the deuterated camptothecin compounds or pharmaceutically acceptable salts thereof, and the use of the deuterated camptothecin compounds, pharmaceutically acceptable salts thereof, or the pharmaceutical compositions in the treatment of tumor-related diseases.

### BACKGROUND

Camptothecin (CPT) is a pyrroloquinoline cytotoxic alkaloid and one of the most extensively studied natural antitumor drugs besides paclitaxel. Camptothecin is primarily found in the fruits and root bark of *Camptotheca acuminata,* a plant in the Nyssaceae family that is unique to China. In 1985, Hsiang *et al.* discovered that camptothecin and its derivatives exert their anticancer effects by targeting topoisomerase I (Topo I) to inhibit DNA synthesis. This revelation reignited widespread interest, leading to the emergence of numerous derivatives and establishing them as a new focal point in anticancer research. Subsequently, a new generation of camptothecin-based drugs, such as 10-hydroxycamptothecin (HCPT), irinotecan, topotecan, SN-38, and belotecan, were successively approved for marketing. These drugs are used to treat cancers such as colorectal cancer, small-cell lung cancer, and ovarian cancer, with promising advancements in both their indications and formulations.

With the development of drug delivery systems, a series of camptothecin drugs that were previously non-drugable or had significant side effects have regained application. However, the issue of *in vivo* toxicity associated with camptothecin drugs has not been entirely avoided. How to further reduce the toxicity of this class of drugs remains an issue that needs attention at present.

### SUMMARY

In one aspect, the present disclosure provides a compound of Formula II, a pharmaceutically acceptable salt thereof, or a stereoisomer thereof: 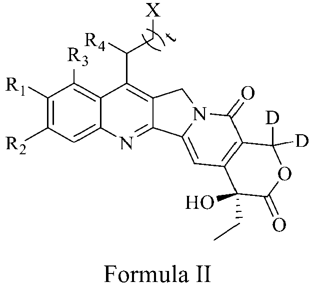
wherein R₁ is H, halogen, OH, SH, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, C₁₋₄ alkoxy, C₁₋₄ haloalkyl, or C₁₋₄ haloalkoxy;
or R₃ and R₄ are cyclized to form -(CH₂)ₖ-, wherein k is 1, 2, 3, or 4;
X is H, OH, HO-CH(R₅)-(CH₂)ₚ-CO-NH-, or -N(R₆)(R₇), wherein p is 0, 1, or 2;
R₅ is H, C₁₋₄ alkyl, C₁₋₄ haloalkyl, 3- to 6-membered cycloalkyl, or 3- to 6-membered heterocycloalkyl;
R₆ is H, C₁₋₄ alkyl, or C₁₋₄ haloalkyl;
R₇ is H or R₈-S(O)₂-;
R₈ is C₁₋₄ alkyl; and
t is 0, 1, 2, 3, 4, or 5.

In the present disclosure, the expression "R₁ and R₂ are cyclized to form -O-(CH₂)ₘ-O-" means that R₁ and R₂ are connected to form -O-(CH₂)ₘ-O-. The expression "R₃ and R₄ are cyclized to form -(CH₂)ₖ-" means that R₃ and R₄ are connected to form -(CH₂)ₖ-.

In any one embodiment of the compound of Formula II of the present disclosure, R₁ is C₁₋₄ alkyl, and R₂ is halogen.

In any one embodiment of the compound of Formula II of the present disclosure, R₁ is methyl, and R₂ is F.

In any one embodiment of the compound of Formula II of the present disclosure, R₁ is H, and R₂ is H.

In any one embodiment of the compound of Formula II of the present disclosure, R₁ and R₂ are cyclized to form -O-CH₂-O-.

In any one embodiment of the compound of Formula II of the present disclosure, R₁ is NH₂, and R₂ is H or halogen. In any one embodiment of the compound of Formula II of the present disclosure, R₁ is NH₂, and R₂ is H or F.

In any one embodiment of the compound of Formula II of the present disclosure, R₃ is H, and R₄ is H.

In any one embodiment of the compound of Formula II of the present disclosure, R₃ is H, and R₄ is C₁₋₄ alkyl.

In any one embodiment of the compound of Formula II of the present disclosure, R₃ is H, and R₄ is methyl.

In any one embodiment of the compound of Formula II of the present disclosure, R₃ and R₄ are cyclized to form -CH₂-CH₂-.

In any one embodiment of the compound of Formula II of the present disclosure, X is HO-CH(R₅)-(CH₂)ₚ-CO-NH-, wherein p is 0, 1, or 2.

In any one embodiment of the compound of Formula II of the present disclosure, X is OH or NH₂.

In any one embodiment of the compound of Formula II of the present disclosure, X is H, and t is 0.

In any one embodiment of the compound of Formula II of the present disclosure, X is - N(R₆)(R₇).

In one embodiment of the compound of Formula II of the present disclosure, the compound of Formula II is a compound of Formula IIa, a pharmaceutically acceptable salt thereof, or a stereoisomer thereof; 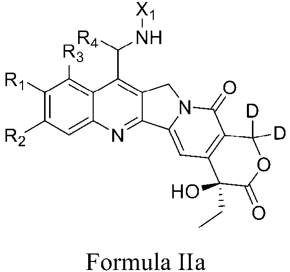 wherein R₁, R₂, R₃, and R₄ are as defined in any one embodiment of the compound of Formula II; X₁ is H or HO-CH(R₅)-(CH₂)ₚ-CO-, wherein p is 0, 1, or 2; R₅ is H, C₁₋₄ alkyl, 3- to 6-membered cycloalkyl, or 3- to 6-membered heterocycloalkyl.

In any one embodiment of the compound of Formula IIa of the present disclosure, R₁ is methyl or methoxy; or R₁ is methyl.

In any one embodiment of the compound of Formula IIa of the present disclosure, R₂ is F or Cl; or R₂ is F.

In any one embodiment of the compound of Formula IIa of the present disclosure, R₁ is methyl, and R₂ is F.

In any one embodiment of the compound of Formula IIa of the present disclosure, R₃ and R₄ are cyclized to form -(CH₂)ₖ-, wherein k is 2.

In any one embodiment of the compound of Formula IIa of the present disclosure, R₃ is H; and R₄ is H.

In any one embodiment of the compound of Formula IIa of the present disclosure, X₁ is HO-CH(R₅)-(CH₂)ₚ-CO-, wherein p is 0 or 1, and R₅ is H, C₁₋₄ alkyl, or 3- to 6-membered cycloalkyl.

In any one embodiment of the compound of Formula IIa of the present disclosure, X₁ is HO-CH(R₅)-(CH₂)ₚ-CO-, wherein p is 0 or 1, and R₅ is H, methyl, or cyclopropyl.

In any one embodiment of the compound of Formula IIa of the present disclosure, X₁ is HO-CH(R₅)-CO-, and R₅ is H or 3- to 6-membered cycloalkyl.

In any one embodiment of the compound of Formula IIa of the present disclosure, X₁ is HO-CH(R₅)-CO-, and R₅ is H or cyclopropyl.

In any one embodiment of the compound of Formula IIa of the present disclosure, X₁ is HO-CH(R₅)-CH₂-CO-, and R₅ is C₁₋₄ alkyl.

In any one embodiment of the compound of Formula IIa of the present disclosure, X₁ is HO-CH(R₅)-CH₂-CO-, and R₅ is methyl.

In any one embodiment of the compound of Formula IIa of the present disclosure, X₁ is the following group: H,

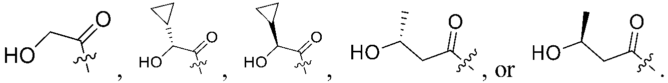

In some embodiments of the compound of Formula IIa of the present disclosure, the compound of Formula IIa is the following compound of Formula IIa-1 or Formula IIa-2, a pharmaceutically acceptable salt thereof, or a stereoisomer thereof; 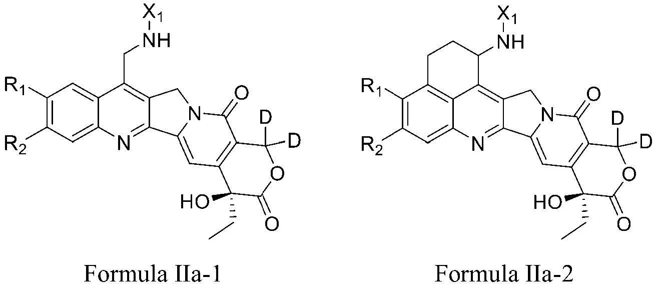 wherein R₁, R₂, and X₁ are as defined in any one embodiment of the compound of Formula IIa.

In some embodiments of the compound of Formula IIa of the present disclosure, the compound of Formula IIa is the following compound, a pharmaceutically acceptable salt thereof, or a stereoisomer thereof:

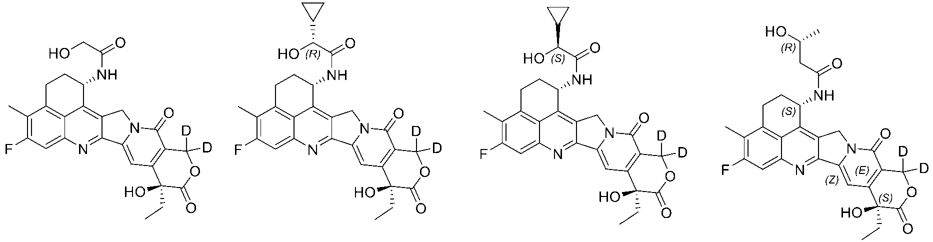

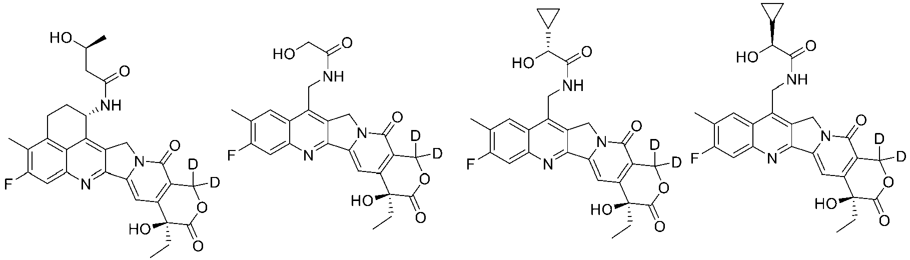

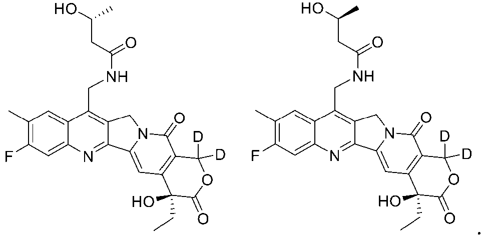

In one embodiment of the compound of Formula II of the present disclosure, the compound of Formula II is a compound of Formula IIb, a pharmaceutically acceptable salt thereof, or a stereoisomer thereof; 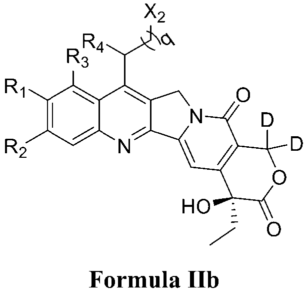
wherein R₁ is H, OH, halogen, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, or C₁₋₄ alkoxy;
or R₃ and R₄ are cyclized to form -(CH₂)ₖ-, wherein k is 1, 2, or 3;
X₂ is H, OH, or -N(R₆)(R₇);
R₆ is H or C₁₋₄ alkyl;
R₇ is H or R₈-S(O)₂-;
R₈ is C₁₋₄ alkyl; and
q is 0, 1, 2, 3, or 4.

In any one embodiment of the compound of Formula IIb of the present disclosure, R₁ is methyl, and R₂ is H, Cl, or F.

In any one embodiment of the compound of Formula IIb of the present disclosure, R₁ and R₂ are cyclized to form -O-(CH₂)ₘ-O-, wherein m is 1 or 2.

In any one embodiment of the compound of Formula IIb of the present disclosure, R₃ and R₄ are both H.

In any one embodiment of the compound of Formula IIb of the present disclosure, R₃ is H, and R₄ is methyl.

In any one embodiment of the compound of Formula IIb of the present disclosure, X₂ is H, OH, NH₂, or N(i-Pr)-S(O)₂-CH₃.

In any one embodiment of the compound of Formula IIb of the present disclosure, X₂ is H, and q is 0; or X₂ is OH or -N(R₆)(R₇), and q is 0, 1, 2, 3, or 4.

In any one embodiment of the compound of Formula IIb of the present disclosure, X₂ is OH or -N(R₆)(R₇); q is 1, 2, 3, or 4; R₃ and R₄ are both H; R₁ is methyl, and R₂ is H, Cl, or F, or R₁ and R₂ are cyclized to form -O-(CH₂)ₘ-O-, wherein m is 1 or 2.

In some embodiments of the compound of Formula IIb of the present disclosure, the compound of Formula IIb is the following compound of Formula IIb-1 or Formula IIb-2; 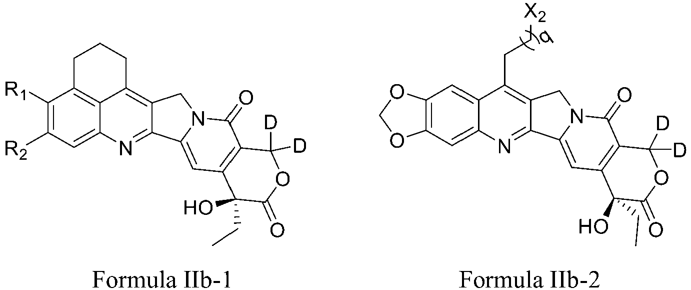 in the compounds of Formula IIb-1 and Formula IIb-2, R₁, R₂, X₂, and q are as defined in any one embodiment of the compound of Formula IIb.

In some embodiments of the compound of Formula IIb, the compound of Formula IIb is the following compound, a pharmaceutically acceptable salt thereof, or a stereoisomer thereof;

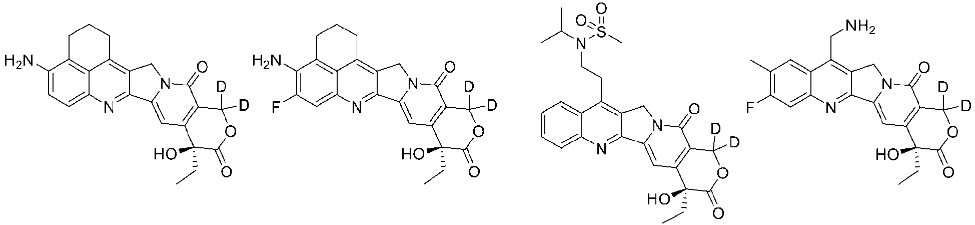

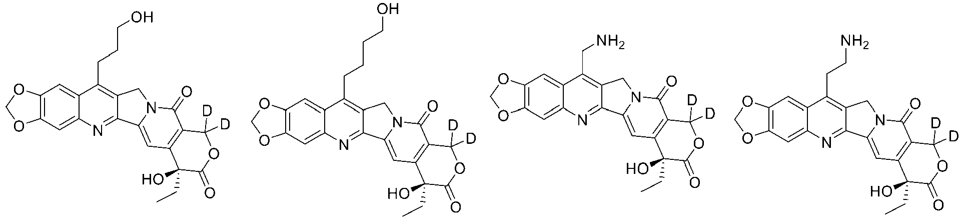

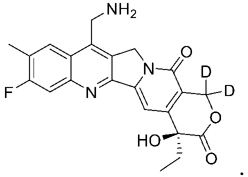

In one embodiment of the present disclosure, the compounds of Formula II, Formula IIa, Formula IIb, Formula IIa-1, Formula IIb-1, Formula IIa-2, and Formula IIb-2 exclude the following compounds;

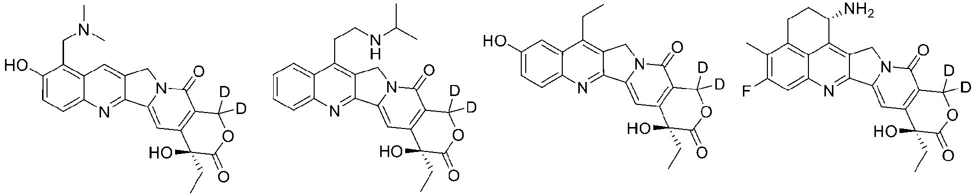

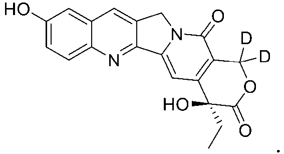

In another aspect, the present disclosure provides a pharmaceutical composition comprising the compound of Formula II, Formula IIa, Formula IIb, Formula IIa-1, Formula IIb-1, Formula IIa-2, or Formula IIb-2, or a pharmaceutically acceptable salt thereof, and a pharmaceutically acceptable excipient.

In yet another aspect, the present disclosure provides the use of the compound of Formula II, Formula IIa, Formula IIb, Formula IIa-1, Formula IIb-1, Formula IIa-2, or Formula IIb-2, or a pharmaceutically acceptable salt thereof, or the pharmaceutical composition in the manufacture of a medicament for treating tumors.

In still another aspect, the present disclosure provides a method for treating tumors, comprising administering to a patient in need thereof an effective amount of the compound of Formula II, Formula IIa, Formula IIb, Formula IIa-1, Formula IIb-1, Formula IIa-2, or Formula IIb-2, or a pharmaceutically acceptable salt thereof, or the pharmaceutical composition.

In still another aspect, the present disclosure provides the compound of Formula II, Formula IIa, Formula IIb, Formula IIa-1, Formula IIb-1, Formula IIa-2, or Formula IIb-2, or a pharmaceutically acceptable salt thereof, or the pharmaceutical composition, for use in treating tumors.

In still another aspect, the present disclosure provides the use of the compound of Formula II, Formula IIa, Formula IIb, Formula IIa-1, Formula IIb-1, Formula IIa-2, or Formula IIb-2, or a pharmaceutically acceptable salt thereof, or the pharmaceutical composition in the treatment of tumors.

It is well known to those skilled in the art that antibody-drug conjugates (ADCs) generally exhibit toxic effects due to the shedding of a small amount of payload *in vivo.* However, the compounds of the present disclosure, particularly Compound 2, show better stability in plasma, shorter half-life *in vivo,* and lower *in vivo* exposure. When used as an ADC payload, they are more readily eliminated upon shedding *in vivo,* resulting in lower toxicity. In addition, toxicity tests of the present disclosure have confirmed that the compounds of the present disclosure exhibit lower *in vivo* toxicity at equivalent doses. Whether used independently as cancer therapeutic agents or as payloads for ADC preparation, they all exhibit improved safety profiles.

### Terms and descriptions

Unless otherwise specified, the terms used herein have the general meanings in the technical field. A specific term or phrase without a particular definition should not be considered indefinite or unclear, but should be understood according to its ordinary meaning. When a trade name appears herein, it is intended to refer to the corresponding trade or its active ingredient.

In any one embodiment of the compound of Formula II of the present disclosure, when R₁ and R₂ are cyclized to form -O-(CH₂)ₘ-O-, the compound of Formula II is the following compound of Formula II-1. In the present disclosure, in Formula II or any one embodiment thereof, when R₃ and R₄ are cyclized to form -(CH₂)ₖ-, the Formula II is presented in the form of the following Formula II-2.

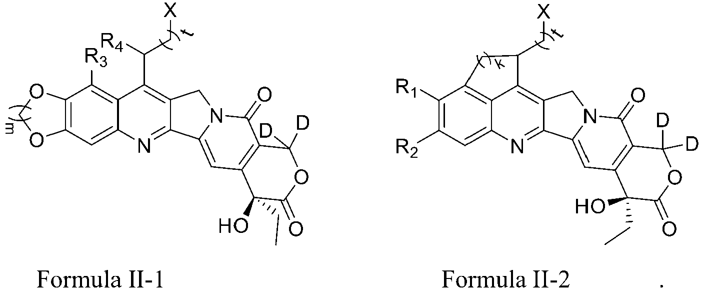

The term "optional" or "optionally" means that the subsequently described event or circumstance may or may not occur, and the description includes instances where the event or circumstance occurs and instances where it does not occur. For example, the term "optionally" substituted means that a group may be unsubstituted or substituted with the designated substituent; whether substituted or not, both are encompassed within the scope of "optionally".

The term "independently" or "each independently" means that each of the aforementioned substituents of the term may select different optional variables from the specified range of choices, and is not influenced by the selection results of other substituents. When a certain substituent that may be "independent" has a plurality of identical substituents in the general chemical formula, it may still choose the same or different optional variables.

The term "halogen" or "halogen atom" refers to fluorine, chlorine, bromine, or iodine.

The term "halo" refers to a group formed by replacing one or more hydrogen atoms in a substituent with halogen atoms.

The term "alkyl" refers to a straight or branched hydrocarbon group where carbon atoms are connected by single bonds. The alkyl is preferably C₁₋₄ or C₁₋₆ alkyl. "C₁₋₄ alkyl" refers to a straight or branched alkyl group having 1, 2, 3, or 4 carbon atoms. Examples of C₁₋₄ alkyl include, but are not limited to, methyl, ethyl, *n*-propyl, isopropyl, *n*-butyl, *sec*-butyl, isobutyl, and *tert*-butyl. "C₁₋₆ alkyl" refers to a straight or branched alkyl group having 1, 2, 3, 4, 5, or 6 carbon atoms. Examples of C₁₋₆ alkyl include, but are not limited to: methyl, ethyl, *n*-propyl, isopropyl, *n*-butyl, *sec*-butyl, isobutyl, *tert*-butyl*, n*-pentyl, and *n*-hexyl.

The term "haloalkyl" refers to an alkyl group in which one or more hydrogen atoms are substituted by halogen atoms. Examples of C₁₋₄ haloalkyl include, but are not limited to, trifluoromethyl, trichloromethyl, 2,2,2-trifluoroethyl, and 2,2,2-trichloroethyl.

The term "alkoxy" refers to alkyl-O-, wherein alkyl is as defined above, including C₁₋₄ alkoxy or C₁₋₆ alkoxy. C₁₋₄ alkoxy can be understood as "C₁₋₄ alkyl-O-", examples of which include, but are not limited to, methoxy, ethoxy, *n*-propoxy, isopropoxy, *n*-butoxy, *sec*-butoxy, and *tert-*butoxy*.* C₁₋₆ alkoxy can be understood as "C₁₋₆ alkyl-O-", examples of which, in addition to the specific examples of C₁₋₄ alkoxy mentioned above, include but are not limited to *n*-pentyloxy, neopentyloxy, *n*-hexyloxy, *etc.*

The term "haloalkoxy" refers to an alkoxy group in which one or more hydrogen atoms are substituted by halogen atoms. Examples of C₁₋₄ haloalkoxy include, but are not limited to, trifluoromethoxy, trichloromethoxy, 2,2,2-trifluoroethoxy, and 2,2,2-trichloroethoxy.

The term "cycloalkyl" refers to a cyclic saturated monocyclic or polycyclic hydrocarbon group in which carbon atoms are connected by single bonds. The hydrogen atoms on the ring carbon atoms may optionally be substituted by oxo, *i.e.,* the "-CH₂-" in the ring may optionally be substituted by oxo to form "-C(O)-". 3- to 6-membered cycloalkyl refers to 3-, 4-, 5-, or 6-membered cycloalkyl, specific examples of which include, but are not limited to, cyclopropyl, cyclobutyl, cyclopentyl, and cyclohexyl.

The term "heterocycloalkyl" refers to a cycloalkyl group in which one or more (*e.g.,* 2, 3, or 4) ring carbon atoms are replaced by a heteroatom or a heteroatom group (*i.e.,* a group containing a heteroatom). The ring carbon atoms refer to the carbon atoms constituting the cyclic skeleton structure; the heteroatom refers to an atom other than C and H in organic compounds, such as nitrogen atoms (N), oxygen atoms (O), sulfur atoms (S), phosphorus atoms (P), or boron atoms (B), wherein the heteroatoms such as nitrogen and sulfur atoms may be oxidized, and the nitrogen atoms may be quaternized; examples of the heteroatom group include but are not limited to -S(=O)₂-, -S(=O)-, and optionally substituted -NH-, -S(=O)(=NH)-, -C(=O)NH-, -C(=NH)-, - S(=O)₂NH-, S(=O)NH-, or -NHC(=O)NH-, *etc.* 3- to 6-membered heterocycloalkyl refers to 3-, 4-, 5-, or 6-membered heterocycloalkyl, with specific examples including but not limited to aziridinyl, oxiranyl, thiiranyl, azetidinyl, oxetanyl, thietanyl, pyrrolidinyl, tetrahydrofuranyl, thiolanyl, piperazinyl, piperidinyl, tetrahydropyranyl, morpholinyl, and 1,4-dioxanyl.

The term "composition" is meant to include a product containing a specified amount of each of the specified ingredients, as well as any product derived directly or indirectly from a combination of the specified amount of each of the specified ingredients. Those skilled in the art can vary the actual dosage levels of the active ingredients in the pharmaceutical compositions of the present disclosure so that the amount of active compound obtained is effective to achieve the desired therapeutic response for a particular patient, composition, and mode of administration.

The term "pharmaceutically acceptable" refers to compounds, materials, compositions and/or dosage forms that are suitable for use in contact with the tissues of humans and animals without excess toxicity, irritation, allergic reactions, or other problems or complications within the scope of reliable medical judgment, and are commensurate with a reasonable benefit/risk ratio.

The term "pharmaceutically acceptable salt" refers to a salt of a pharmaceutically acceptable acid or base, including salts formed by the compound with inorganic or organic acids, as well as salts formed by the compound with inorganic or organic bases.

The term "excipient" generally refers to carriers, diluents, and/or media required for formulating an effective pharmaceutical composition.

The term "effective amount" refers to an amount of the compound of the present disclosure or a pharmaceutically acceptable salt thereof that is suitable for treating a disease or condition with a reasonable benefit/risk ratio applicable to any medical treatment. The amount of the compound of the present disclosure that constitutes an "effective amount" varies depending on the compound, the disease condition and its severity, the route of administration, and the age of the mammal to be treated, but can be routinely determined by a person skilled in the art based on their own knowledge and the content of the present disclosure.

In the present disclosure, the " 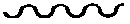 " in a substituent indicates the attachment position of the substituent to the parent structure or other fragment. The appearance of a dash "-" in a substituent structure is used to indicate the attachment point of the substituent. For example, - CH₃ indicates that the group is connected to the parent structure or other fragment through the C atom. " 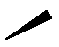 " and " 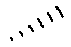 " denote the absolute configuration of a stereocenter, *i.e., R* or *S* configuration.

In the present disclosure, the chemical bond depicted by the solid and dashed line "- - - - - - " represents a single or double bond, which can be determined within the scope commonly known to those skilled in the art based on the valences of the atoms at both ends of the chemical bond.

In the present disclosure, the term "isomer" includes geometric isomers and stereoisomers, such as atropisomers, *cis-trans* isomers, enantiomers, diastereomers, tautomers, and their racemic mixtures and other mixtures, all of which fall within the scope of the present disclosure. The term "enantiomer" refers to stereoisomers that are mirror images of each other. The term "tautomer" refers to a class of functional group isomers having different points of hydrogen attachment via displacement of one or more double bonds, for example, a ketone and its enol form are keto-enol tautomers. The term "diastereomer" refers to a stereoisomer where the molecule has two or more chiral centers and the molecules are not mirror images of each other. The term *"cis-trans* isomer" refers to different spatial configurations in which double bonds or single bonds of ring carbon atoms in a molecule cannot rotate freely. The term "atropisomer" refers to stereoisomers that can be separated due to hindered or extremely slow rotation of single bonds.

The stereoisomers of the compounds of the present disclosure may be prepared by chiral synthesis, chiral reagents, or other conventional techniques. For example, one enantiomer of a certain compound of the present disclosure may be prepared by asymmetric catalytic techniques or chiral auxiliary derivatization techniques. Alternatively, a single stereoisomeric compound can be obtained from a mixture via chiral resolution techniques. Alternatively, a single stereoisomeric compound can be prepared directly using chiral starting materials. The separation of optically pure compounds in the present disclosure is typically accomplished by preparative chromatography using a chiral chromatographic column to achieve the separation of chiral compounds.

In the present disclosure, the solution concentration unit M represents mol/L, mM represents mmol/L, and nM represents nmol/L. The solution concentration unit N represents equivalent concentration, which is expressed as the number of gram equivalents of solute contained in 1 liter of solution, denoted by the symbol N. For example, if 1 liter of concentrated hydrochloric acid contains 12.0 gram equivalents of hydrochloric acid (HCl), its concentration is 12.0 N. Equivalent concentration = number of gram equivalents of solute/volume of solution (L).

The chemical abbreviations used in the present disclosure and their corresponding chemical names are as follows:

| **Chemical abbreviation** | **Chemical name** |
|---|---|
| DBU | 1,8-Diazabicyclo[5.4.0]undec-7-ene |
| HOBT | 1-Hydroxybenzotriazole |
| EDCI | Carbodiimide |
| DIPEA | Diisopropylethylamine |
| TCEP | Tris(2-carboxyethyl)phosphine hydrochloride |
| DME | Ethylene glycol dimethyl ether |
| DMTMM | 4-(4,6-Dimethoxy-1,3,5-triazin-2-yl)-4-methylmorpholinium chloride |
| MTBE | Methyl *tert-*butyl ether |

### Example

The present disclosure is further illustrated in detail below through specific preparation examples and biological experiments. However, it should be understood that these examples and biological experiments are provided solely for illustrative purposes and should not be construed as limiting the present disclosure in any form. It is clear to those skilled in the art that, unless otherwise specified, the materials used herein are well-known in the field, can be purchased commercially, or obtained by those skilled in the art based on published literature or conventional methods. Unless otherwise stated, all reactions in the present disclosure are carried out under continuous magnetic stirring in a dry nitrogen or argon atmosphere, using dry solvents, wherein: (i) temperatures are given in degrees Celsius (°C), and operations are performed at room temperature, which generally refers to 15-35°C, preferably 20-30°C, and more preferably 20-25°C; (ii) solvent removal is achieved by reduced-pressure evaporation using a rotary evaporator, with a bath temperature not exceeding 60°C; (iii) reaction progress is monitored by thin-layer chromatography (TLC); (iv) final products exhibit satisfactory hydrogen nuclear magnetic resonance spectroscopy (¹H-NMR) and/or mass spectrometry (MS) data.

### Example 1: Preparation of Compound 1

### Step 1:

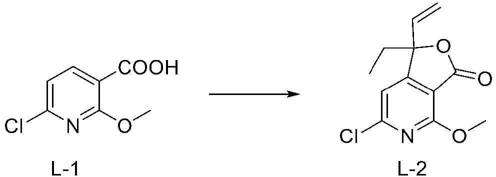

86.4 mL of 2,2,6,6-tetramethylpiperidine was added to a 3 L three-necked flask, followed by the addition of 1 L of anhydrous tetrahydrofuran. The system was cooled to -78°C, and the reaction was carried out for 15 minutes. 272 mL of a 2.5 M n-butyllithium solution in petroleum ether was added, and the reaction was carried out for 30 minutes. 32 g of compound L-1 was dissolved in 200 mL of anhydrous tetrahydrofuran, then added to the system, and the reaction was carried out for 1 hour. 50 mL of 1-penten-3-one was dissolved in 200 mL of anhydrous tetrahydrofuran and then added to the system. The reaction was carried out for 1 hour. After completion of the reaction (monitored by TLC), 640 mL of 4 N hydrochloric acid was added, and the mixture was extracted with ethyl acetate. The organic phases were combined, washed with saturated aqueous sodium bicarbonate solution, washed with saturated brine, and dried over anhydrous sodium sulfate. After the system was concentrated under vacuum, 21 g of compound L-2 was obtained by column chromatography purification, with a yield of 49%. ESI-MS m/z: 253.68, [M+H]⁺.

¹H NMR (400 MHz, CDCl₃) δ 6.99 (s, 1H), 6.13 (dd, J = 17.3, 10.6 Hz, 1H), 5.29 (d, J = 7.0 Hz, 1H), 5.26 (s, 1H), 3.99 (s, 3H), 2.73 (s, 2H), 2.04 (dt, J = 14.8, 7.3 Hz, 1H), 1.98 - 1.87 (m, 1H), 0.91 (t, J = 7.3 Hz, 3H).

### Step 2:

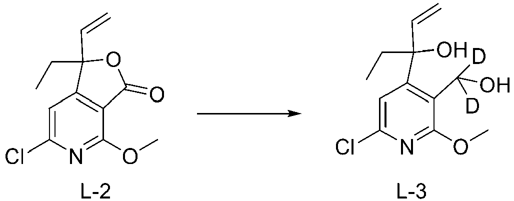

21 g of compound L-2 was added to a 1 L three-necked flask, followed by the addition of 400 mL of anhydrous ethanol. The system was cooled to 0°C, and then 14.21 g of sodium borodeuteride was added. The reaction mixture was stirred for 15 minutes and then warmed to room temperature to react for 12 hours. After completion of the reaction (monitored by TLC), 30 mL of 1 N hydrochloric acid was added, and the mixture was extracted with dichloromethane. The organic phases were combined, washed successively with saturated aqueous sodium bicarbonate solution and saturated brine, and dried over anhydrous sodium sulfate. After the system was concentrated under vacuum, 17 g of compound L-3 was obtained by column chromatography purification as a transparent viscous liquid, with a yield of 80%. ESI-MS m/z: 259.73, [M+H]⁺. ¹H NMR (400 MHz, CDCl₃) δ 6.99 (s, 1H), 6.13 (dd, J = 17.3, 10.6 Hz, 1H), 5.29 (d, J = 7.0 Hz, 1H), 5.26 (s, 1H), 3.99 (s, 3H), 2.73 (s, 2H), 2.04 (dt, J = 14.8, 7.3 Hz, 1H), 1.98 - 1.87 (m, 1H), 0.91 (t, J = 7.3 Hz, 3H).

### Step 3:

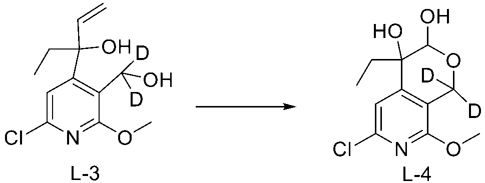

2 g of compound L-3 was added to a 250 mL round-bottom flask, followed by the addition of 100 mL of dichloromethane. The system was cooled to -78°C, and ozone was introduced under these conditions. After completion of the reaction (monitored by TLC), 0.5 mL of dimethyl sulfide was added, and the reaction mixture was warmed to room temperature and stirred for 30 minutes. After the system was concentrated under vacuum, 1.9 g of compound L-4 was obtained by column chromatography purification, with a yield of 95%. The product was directly used in the next step without further purification. ESI-MS m/z: 261.70, [M+H]⁺.

¹H NMR (400 MHz, CDCl₃) δ 7.15 (s, 1H), 5.19 (d, J = 4.8 Hz, 1H), 3.96 (s, 3H), 3.21 (d, J = 4.8 Hz, 1H), 2.64 (s, 1H), 1.80 (q, J = 7.5 Hz, 2H), 0.92 (t, J = 7.5 Hz, 3H).

### Step 4:

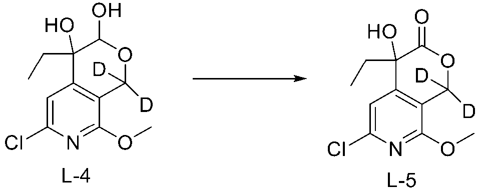

12 g of compound L-4 was added to a 1 L single-necked flask, followed by the addition of 240 mL of dichloromethane. The mixture was stirred for 30 minutes, and the system was cooled to 0°C. 286 mg of 2,2,6,6-tetramethylpiperidine oxide, 616 mg of sodium bicarbonate, 654 mg of potassium bromide, and 18 mL of water were added, and the reaction mixture was stirred for 15 minutes. 120 mL of aqueous sodium hypochlorite solution (>7.5wt%) was added, and the reaction was carried out for 30 minutes. After completion of the reaction (monitored by TLC), 12 g of sodium bisulfite was added, and the mixture was extracted with dichloromethane. The organic phases were combined, washed with water, washed with saturated brine, and dried over anhydrous sodium sulfate. After the system was concentrated under vacuum, 6.6 g of compound L-5 was obtained by column chromatography purification, with a yield of 55%. ESI-MS m/z: 259.68, [M+H]⁺.

¹H NMR (400 MHz, CDCl₃) δ 7.20 (s, 1H), 4.00 (s, 3H), 3.64 (s, 1H), 1.79 (q, J = 7.4 Hz, 2H), 0.97 (t, J = 7.4 Hz, 3H).

### Step 5:

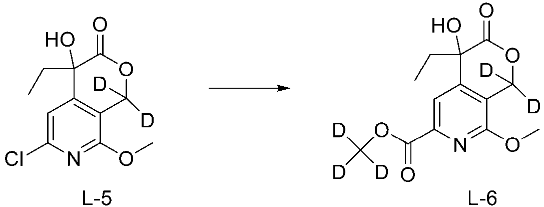

Under a carbon monoxide atmosphere, 3 g of compound L-5 was added to a 100 mL three-necked flask, followed by the addition of 286 mg of 1,3-bis(diphenylphosphino)propane, 2.4 g of potassium carbonate, 129 mg of palladium acetate, 15 mL of *N,N-*dimethylformamide, and 30 mL of deuterated methanol. The system was heated to 65°C, and the reaction was carried out for 12 hours. After completion of the reaction (monitored by TLC), 20 mL of 1 N hydrochloric acid was added, and the mixture was extracted with ethyl acetate. The organic phases were combined, washed successively with saturated aqueous sodium bicarbonate solution and saturated brine, and dried over anhydrous sodium sulfate. After the system was concentrated under vacuum, 1.33 g of compound L-6 was obtained by column chromatography purification, with a yield of 40%. ESI-MS m/z: 286.29, [M+H]⁺.

¹H NMR (400 MHz, CDCl₃) δ 7.98 (d, J = 2.0 Hz, 1H), 4.09 (d, J = 2.0 Hz, 3H), 3.71 (d, J = 2.6 Hz, 1H), 1.88 - 1.75 (m, 2H), 0.96 (td, J = 7.3, 1.9 Hz, 3H).

### Step 6:

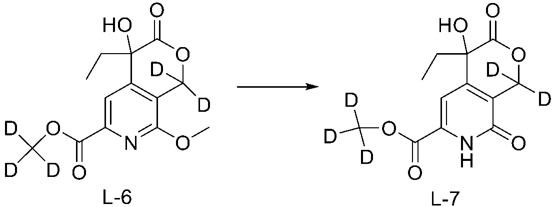

1 g of compound L-6 was added to a 50 mL two-necked flask, followed by the addition of 1.05 g of sodium iodide and 10 mL of acetonitrile. The system was cooled to 0°C and stirred for 30 minutes. 0.89 mL of trimethylsilyl chloride was added, and the system was warmed to room temperature to react for 12 hours. After completion of the reaction (monitored by TLC), 40 mL of water and 1 mL of saturated aqueous sodium bisulfite solution were added, and the mixture was stirred for 1 hour. The mixture was extracted with dichloromethane and dried over anhydrous sodium sulfate. After the system was concentrated under vacuum, 800 mg of compound L-7 was obtained by column chromatography purification, with a yield of 84%. ESI-MS m/z: 272.27, [M+H]⁺.

¹H NMR (400 MHz, CDCl₃) δ 10.03 (s, 1H), 7.32 (s, 1H), 3.90 (s, 1H), 1.81 (dd, J = 14.5, 7.2 Hz, 2H), 0.99 (t, J = 7.4 Hz, 3H).

### Step 7:

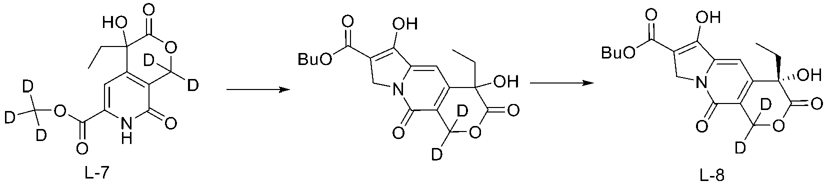

620 mg of compound L-7 was added to a 25 mL two-necked flask, followed by the addition of 1.48 g of cesium carbonate, 8 mL of dimethyl sulfoxide, and 1.67 mL of *tert-*butyl acrylate. The system was heated to 50°C, and the reaction was carried out for 12 hours. After completion of the reaction (monitored by TLC), 1 mL of concentrated hydrochloric acid was added, followed by the addition of 40 mL of water. The mixture was extracted with dichloromethane and dried over anhydrous sodium sulfate. After the system was concentrated under vacuum, 468 mg of compound L-8 was obtained by preparative SPC chiral separation (instrument model: SFC-350 (Waters), chromatographic column: AS 25 * 250 mm, 10 µm, mobile phase: CO₂/MeOH = 65/35, flow rate: 200 mL/min), with a yield of 55%. ESI-MS m/z: 365.38, [M+H]⁺.

### Step 8:

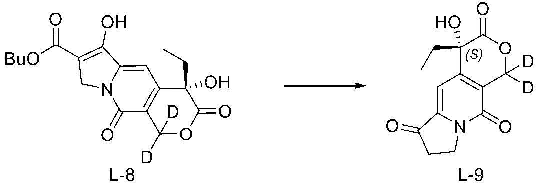

200 mg of compound L-8 was added to a 10 mL single-necked flask, followed by the addition of 5 mL of toluene and 0.5 mL of trifluoroacetic acid. The system was heated to 110°C, and the reaction was carried out for 2 hours. After completion of the reaction (monitored by TLC), the system was concentrated under vacuum, and 140 mg of compound L-9 was obtained by column chromatography purification, with a yield of 98%. ESI-MS m/z: 265.26, [M+H]⁺.

### Step 9:

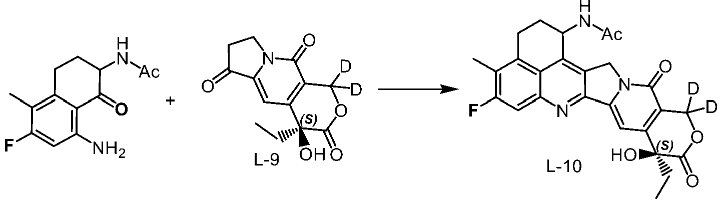

300 mg of compound L-9 was added to a 25 mL single-necked flask, followed by the addition of 266 mg of *N*-(5-methyl-6-fluoro-8-amino-1-oxo-1,2,3,4-tetrahydronaphthalen-2-yl)acetamide, 9 mL of toluene, 770 mL of o-cresol, and 133 mg of pyridinium *p*-toluenesulfonate. The system was heated to 110°C, and the reaction was carried out for 18 hours. After completion of the reaction (monitored by TLC), the system was concentrated under vacuum, and 443 mg of compound L-10 was obtained by column chromatography purification (dichloromethane: methanol = 10:1), with a yield of 81%.

### Step 10:

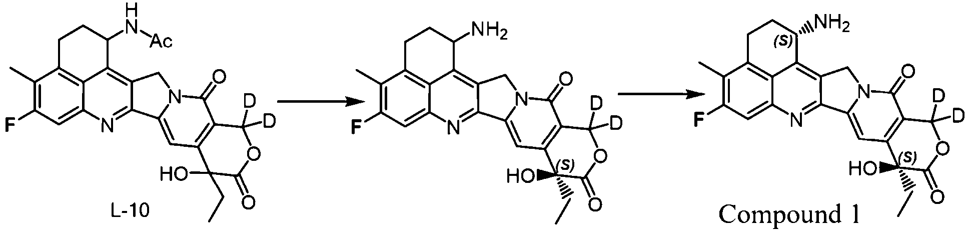

443 mg of intermediate compound L-10 was added to a 25 mL single-necked flask, followed by the addition of 7 mL of purified water and 2.2 mL of methanesulfonic acid. The system was purged with nitrogen. The system was heated to 85°C, and the reaction was carried out for 10 hours. After completion of the reaction (monitored by TLC), the system was concentrated under vacuum, and 141 mg of compound 1 was obtained by column chromatography purification (dichloromethane: methanol), with a yield of 35%. ESI-MS m/z: 437.47, [M+H]⁺.

¹H NMR (400 MHz, D₂O) δ 7.20 - 7.06 (m, 2H), 5.42 - 5.14 (m, 5H), 3.30 (dd, J = 18.1, 4.0 Hz, 1H), 3.06 - 2.91 (m, 1H), 2.69 (s, 2H), 2.62 - 2.47 (m, 1H), 2.18 (s, 3H), 1.80 (q, J = 7.3 Hz, 2H), 0.79 (t,J = 7.4 Hz, 3H).

### Example 2: Preparation of Compound 2

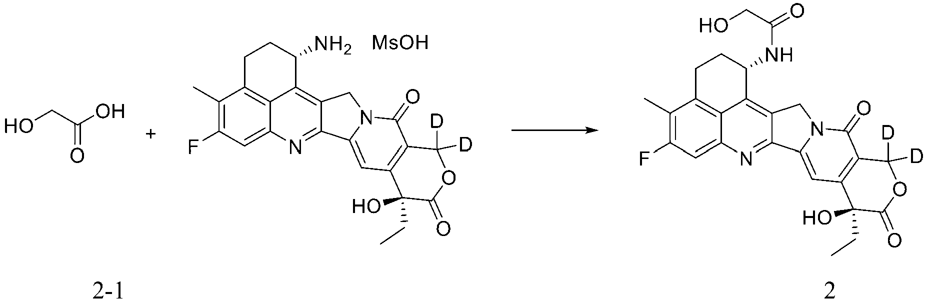

2.4 mL of water and 3.0 mL of tetrahydrofuran were added to a mixture of anhydrous sodium sulfate (2.25 eq), ethyl 2-cyano-2-hydroxyiminoacetate (2.25 eq), 2-hydroxyacetic acid (1.45 eq), and the methanesulfonate salt of compound 1 (100 mg). After stirring at room temperature for 15 minutes, *N*-methylmorpholine (1.10 eq) was added, and the mixture was stirred at room temperature for 15 minutes. Subsequently, EDCI (2 eq) was added, and the reaction mixture was stirred at room temperature for 3 hours. After the complete reaction of compound 1 was monitored by HPLC, the reaction mixture was filtered, and the filter cake was dried. The title compound was obtained by silica gel column chromatography purification (DCM: MeOH).

¹H NMR (400 MHz, DMSO) δ 8.41 (d, *J =* 8.9 Hz, 1H), 7.77 (d, *J =* 11.0 Hz, 1H), 7.30 (s, 1H), 6.52 (s, 1H), 5.58 (d, *J =* 5.4 Hz, 1H), 5.49 (t, *J =* 5.7 Hz, 1H), 5.20 (t, *J =* 13.9 Hz, 2H), 3.96 (d, *J =* 5.5 Hz, 2H), 3.28 - 3.05 (m, 2H), 2.39 (s, 3H), 2.29 - 2.11 (m, 2H), 1.96 - 1.78 (m, 2H), 0.87 (t, *J* = 7.2 Hz, 3H). MS (ESI) m/z [M+H]⁺: 496.1.

### Example 3: Preparation of Compound 3

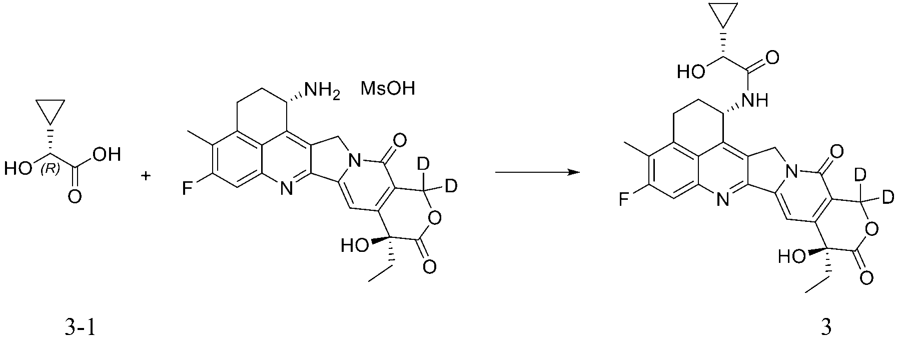

Referring to the preparation method of Example 2, 52 mg of compound 3 was prepared using (*R*)-2-cyclopropyl-2-hydroxyacetic acid as the starting material, with a yield of 52%.

¹H NMR (400 MHz, DMSO-*d*₆) δ 8.36 (d, J = 8.4 Hz, 1H), 7.78 (d, J = 10.9 Hz, 1H), 7.31 (s, 1H), 6.52 (s, 0H), 5.58 - 5.48 (m, 1H), 5.38 (d, J = 5.3 Hz, 1H), 5.21 (q, J = 19.0 Hz, 2H), 3.62 (d, J = 5.2 Hz, 1H), 3.16 (dd, J = 11.2, 5.7 Hz, 3H), 2.39 (s, 4H), 2.22 - 2.12 (m, 3H), 1.86 (dt, J = 14.3, 7.1 Hz, 2H), 1.14 (q, J = 7.3 Hz, 2H), 0.87 (t, J = 7.2 Hz, 3H), 0.38 (dd, J = 20.9, 5.6 Hz, 4H). MS (ESI) m/z [M+H]⁺: 536.2.

### Example 4: Preparation of Compound 4

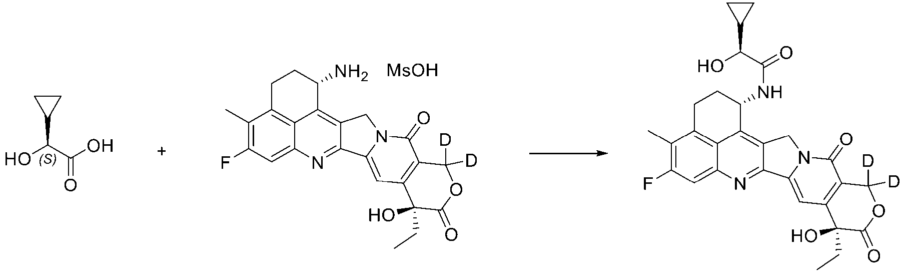

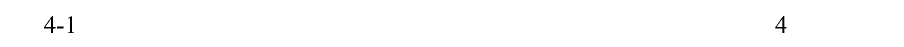

Referring to the preparation method of Example 2, 54 mg of compound 4 was prepared using (*S*)-2-cyclopropyl-2-hydroxyacetic acid as the starting material, with a yield of 54%.

¹H NMR (400 MHz, DMSO-*d*₆) δ 8.37 (d, *J =* 9.0 Hz, 1H), 7.77 (d, *J =* 11.0 Hz, 1H), 7.30 (s, 1H), 6.51 (s, 1H), 5.58 (q, *J =* 6.7 Hz, 1H), 5.49 (d, *J =* 5.2 Hz, 1H), 5.35 - 5.06 (m, 2H), 3.60 (t, *J =* 5.7 Hz, 1H), 3.17 (q, *J =* 17.2 Hz, 2H), 2.39 (s, 3H), 2.16 (d, *J =* 6.1 Hz, 2H), 1.85 (dq, *J =* 14.2, 7.1 Hz, 2H), 1.24 (q, *J =* 8.1 Hz, 1H), 0.87 (t, *J =* 7.3 Hz, 3H), 0.59 - 0.32 (m, 4H). MS (ESI) m/z [M+H]⁺: 536.2.

### Example 5: Preparation of Compound 5

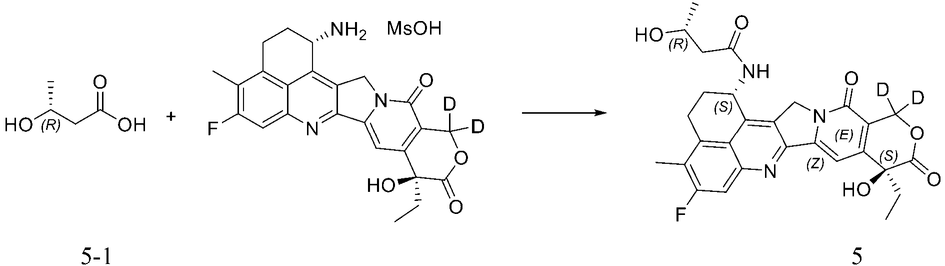

Referring to the preparation method of Example 2, 55 mg of compound 5 was prepared using (*R*)-3-hydroxybutyric acid as the starting material, with a yield of 56%.

¹H NMR (400 MHz, DMSO-*d*₆) δ 8.42 (d, J = 8.7 Hz, 1H), 7.78 (d, J = 11.0 Hz, 1H), 7.30 (s, 1H), 6.52 (s, 1H), 5.55 (dt, J = 9.3, 5.0 Hz, 1H), 5.29 - 5.12 (m, 2H), 4.66 (d, J = 4.7 Hz, 1H), 4.05 (dt, J = 12.1, 6.0 Hz, 1H), 3.25 - 3.09 (m, 2H), 2.39 (s, 3H), 2.33 - 2.17 (m, 2H), 2.13 (dd, J = 10.7, 5.4 Hz, 2H), 1.86 (hept, J = 7.1 Hz, 2H), 1.09 (d, J = 6.2 Hz, 3H), 0.87 (t, J = 7.3 Hz, 3H). MS (ESI) m/z [M+H]⁺: 524.2.

### Example 6: Preparation of Compound 6

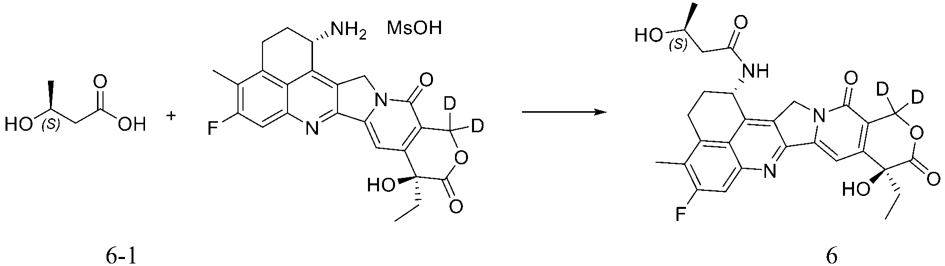

Referring to the preparation method of Example 2, 51 mg of compound 6 was prepared using (*S*)-3-hydroxybutyric acid as the starting material, with a yield of 52%.

¹H NMR (400 MHz, DMSO-*d*₆) δ 8.41 (d, *J =* 8.7 Hz, 1H), 7.78 (d, *J =* 11.0 Hz, 1H), 7.30 (s, 1H), 6.53 (s, 1H), 5.57 (dt, *J =* 8.8, 4.5 Hz, 1H), 5.22 (d, *J =* 2.7 Hz, 2H), 4.64 (d, *J =* 4.5 Hz, 1H), 4.05 (dt, *J =* 11.9, 6.1 Hz, 1H), 3.16 (t, *J =* 5.7 Hz, 2H), 2.39 (s, 3H), 2.32 - 2.17 (m, 2H), 2.17 - 2.04 (m, 2H), 1.86 (hept, *J* = 7.1 Hz, 2H), 1.08 (d, *J =* 6.1 Hz, 3H), 0.87 (t, *J =* 7.3 Hz, 3H). MS (ESI) m/z [M+H]⁺: 524.2.

### Example 7: Preparation of Compound 7

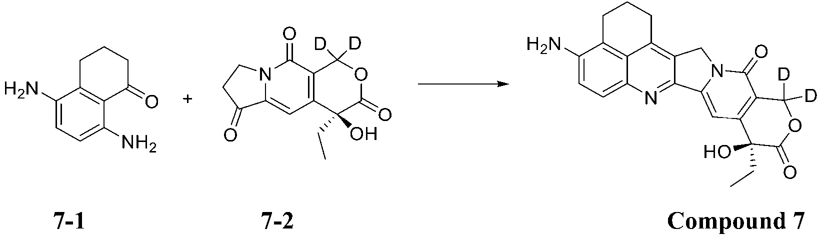

Compound 7-1 (87 mg, 1 eq), compound 7-2 (144 mg, 1.1 eq), pyridinium *p-*toluenesulfonate (50 mg, 0.4 eq), acetic acid (2.6 mL, 30 V), and toluene (2.6 mL, 30 V) were added to a reaction flask. The reaction was carried out at 110°C for 18 hours. After completion of the reaction (monitored by TLC), the solvent was removed by rotary evaporation. 123 mg of compound 7 was obtained by silica gel column chromatography purification (dichloromethane: methanol), with a yield of 61%.

¹H NMR (400 MHz, DMSO-*d*₆) δ 7.72 (d, J = 9.0 Hz, 1H), 7.29 (d, J = 9.0 Hz, 1H), 7.17 (s, 1H), 6.45 (s, 1H), 5.65 (s, 2H), 5.16 (s, 2H), 3.04 (t, J = 6.1 Hz, 2H), 2.75 (t, J = 6.1 Hz, 2H), 2.08 - 1.95 (m, 2H), 1.85 (hept, J = 7.1 Hz, 2H), 0.88 (t, J = 7.2 Hz, 3H). MS (ESI) m/z [M+H]⁺: 406.1.

### Example 8: Preparation of Compound 8

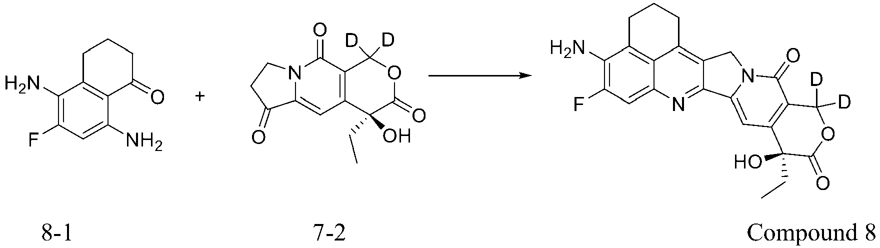

Referring to the preparation method of Example 7, 127 mg of compound 8 was prepared using compounds 8-1 and 7-2 as the starting materials, with a yield of 60%.

¹H NMR (400 MHz, DMSO-*d*₆) δ 8.41 (d, *J =* 8.7 Hz, 1H), 7.78 (d, *J =* 11.0 Hz, 1H), 7.30 (s, 1H), 6.53 (s, 1H), 5.57 (dt, *J =* 8.8, 4.5 Hz, 1H), 5.22 (d, *J=* 2.7 Hz, 2H), 4.64 (d, *J =* 4.5 Hz, 1H), 4.05 (dt, *J =* 11.9, 6.1 Hz, 1H), 3.16 (t, *J =* 5.7 Hz, 2H), 2.39 (s, 3H), 2.32 - 2.17 (m, 2H), 2.17 - 2.04 (m, 2H), 1.86 (hept, *J* = 7.1 Hz, 2H), 1.08 (d, *J =* 6.1 Hz, 3H), 0.87 (t, *J =* 7.3 Hz, 3H). MS (ESI) m/z [M+H]⁺: 424.1.

### Example 9: Preparation of Compound 9

### Step 1:

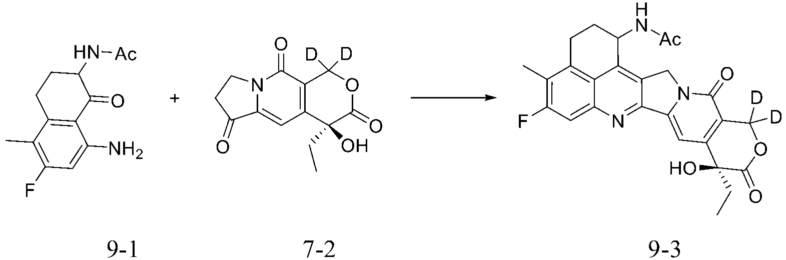

250 mg of compound 9-1, 300 mg of compound 7-2, 7.5 mL of acetic acid, 7.5 mL of toluene, and 68 mg of pyridinium *p*-toluenesulfonate were added to a 50 mL single-necked flask. The reaction was carried out at 110°C for 18 hours. After completion of the reaction (monitored by TLC), the solvent was evaporated under reduced pressure, and 386 mg of compound 9-3 was obtained by silica gel column chromatography purification (DCM: MeOH), with a yield of 81%. MS (ESI) m/z [M+H]⁺: 480.2.

### Step 2:

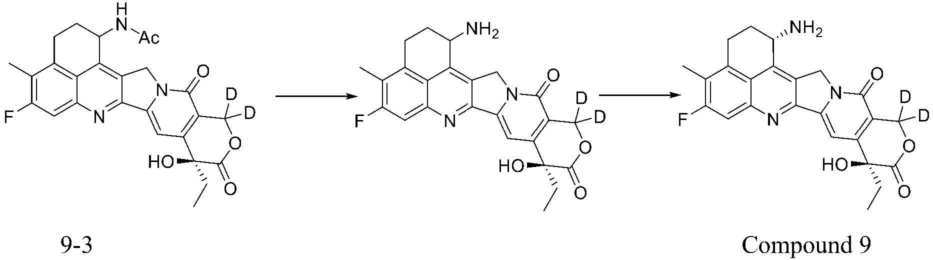

380 mg of compound 9-3 was added to a 100 mL single-necked flask, followed by the addition of 7.6 mL of purified water and 3.8 mL of methanesulfonic acid. The reaction was carried out at 85°C for 10 hours under a nitrogen atmosphere. After completion of the reaction (monitored by TLC), the reaction mixture was cooled to room temperature, and 22.8 mL of methanol was added. The mixture was stirred at room temperature for 2 hours, filtered, and the crude product was obtained. The crude product was purified by silica gel column chromatography (dichloromethane: methanol) to obtain 148 mg of compound 9, with a yield of 35%.

1H NMR (400 MHz, D2O) δ 7.20-7.06 (m, 2H), 5.42-5.14 (m, 5H), 3.30 (dd, J = 18.1, 4.0 Hz, 1H), 3.06-2.91 (m, 1H), 2.69 (s, 2H), 2.62-2.47 (m, 1H), 2.18 (s, 3H), 1.80 (q, J = 7.3 Hz, 2H), 0.79 (t, J = 7.4 Hz, 3H). MS (ESI) m/z [M+H]⁺: 438.2.

### Example 10: Preparation of Compound 10

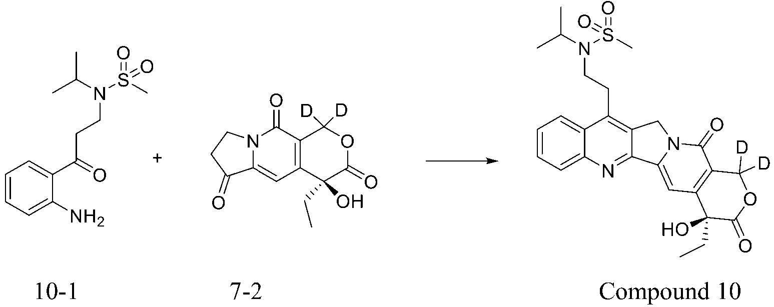

Referring to the preparation method of Example 9, 70 mg of compound 10 was prepared using compound 10-1 and compound 7-2 as the starting materials, with a yield of 34%.

¹H NMR (400 MHz, DMSO-*d*₆) δ 8.32 (d, J = 8.4 Hz, 1H), 8.20 (dd, J = 8.4, 1.2 Hz, 1H), 7.93-7.84 (m, 1H), 7.79 (t, J = 7.6 Hz, 1H), 7.35 (s, 1H), 6.56 (s, 1H), 5.42 (s, 2H), 3.98 (p, J = 6.7 Hz, 1H), 3.50 (t, J = 8.0 Hz, 2H), 3.42-3.35 (m, 2H), 3.00 (s, 3H), 1.88 (hept, J = 7.3 Hz, 2H), 1.15 (d, J = 6.7 Hz, 6H), 0.88 (t, J = 7.3 Hz, 3H). MS (ESI) m/z [M+H]⁺: 514.2.

### Example 11: Preparation of Compound 11

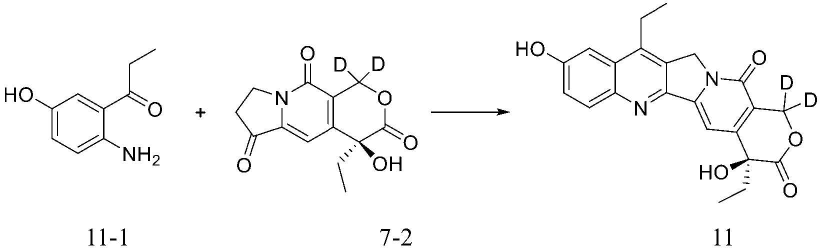

Referring to the preparation method of Example 9, 80 mg of compound 11 was prepared using compound 11-1 and compound 7-2 as the starting materials, with a yield of 37%.

¹H NMR (500 MHz, DMSO-*d*₆) δ 0.88 (t, J = 7.25 Hz, 3H),1.32 (t, J = 7.5 Hz, 3H), 1.85 (m, 2H), 3.11 (q, J = 7.5 Hz, 2H), 5.26(s, 2H), 6.48(s, 1H), 7.23 (s, 1H), 7.41 (d, J = 10.0 Hz, 2H), 8.01 (d, J = 10.0 Hz, 1H), 10.3 (s, 1H). MS (ESI) m/z [M+H]⁺: 395.1.

### Example 12: Preparation of Compound 12

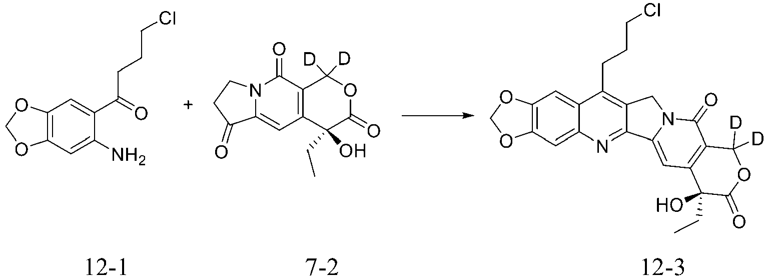

255 mg of compound 12-1, 300 mg of compound 7-2, 7.5 mL of acetic acid, 7.5 mL of toluene, and 68 mg of pyridinium *p*-toluenesulfonate were added to a 50 mL single-necked flask. The reaction was carried out at 110°C for 18 hours. After completion of the reaction (monitored by TLC), the solvent was evaporated under reduced pressure, and 330 mg of compound 12-3 was obtained by silica gel column chromatography purification (DCM: MeOH), with a yield of 70%.

¹H NMR (400 MHz, DMSO-*d*₆) δ 7.63 (s, 1H), 7.51(s, 1H), 7.24 (s, 1H), 6.50 (s, 1H), 6.30 (s, 2H), 5.26 (s, 2H), 3.81 (d, J = 5.9 Hz, 2H), 3.22 (s, 2H), 1.98 (d, J = 6.7 Hz, 4H), 0.88 (t, J = 7.2 Hz, 3H). MS (ESI) m/z [M+H]⁺: 471.1.

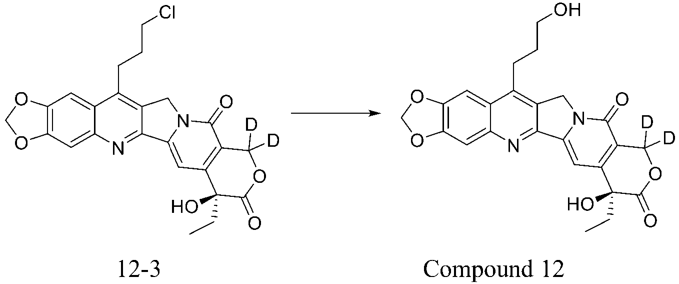

300 mg of intermediate compound 12-3 was dissolved in 15 mL of 10% sulfuric acid, and the reaction was carried out at 110°C for 48 hours. After completion of the reaction (monitored by TLC), the mixture was neutralized by adding a saturated aqueous sodium carbonate solution, extracted with dichloromethane, dried over anhydrous sodium sulfate, filtered, and concentrated under reduced pressure to obtain a crude product. The crude product was purified by silica gel column chromatography (DCM: MeOH) to obtain 89 mg of compound 12, with a yield of 31%.

¹H NMR (400 MHz, DMSO-*d*₆) δ 7.63 (s, 1H), 7.50(s, 1H), 7.24 (s, 1H), 6.48 (s, 1H), 6.28 (s, 2H), 5.32-5.19 (m, 2H), 3.51-3.46 (m, 2H), 3.17-3.13 (m, 2H), 1.92 - 1.76 (m, 4H), 0.88 (t, J = 7.2 Hz, 3H). MS (ESI) m/z [M+H]⁺: 453.2.

### Example 13: Preparation of Compound 13

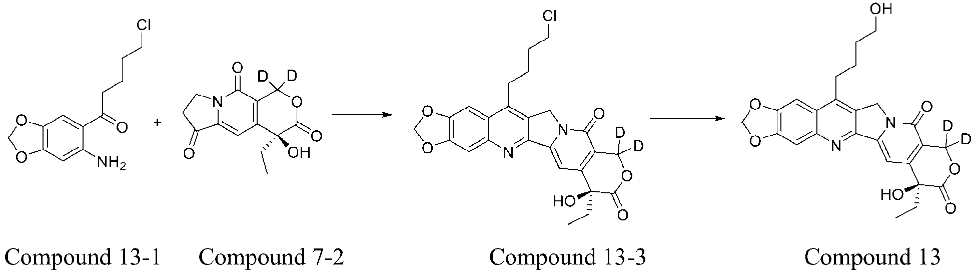

Referring to the preparation method of Example 12, 74 mg of compound 13 was prepared using compound 13-1 and compound 7-2 as the starting materials, with a two-step yield of 16%.

¹H NMR (400 MHz, DMSO-*d*₆) δ 7.63 (s, 1H), 7.50(s, 1H), 7.24 (s, 1H), 6.48 (s, 1H), 6.28 (s, 2H), 5.32-5.19 (m, 2H), 3.51-3.46 (m, 2H), 3.17-3.13 (m, 2H), 1.94 - 1.76 (m, 6H), 0.88 (t, J = 7.2 Hz, 3H). MS (ESI) m/z [M+H]⁺: 467.2.

### Example 14: Preparation of Compound 14

### Step 1:

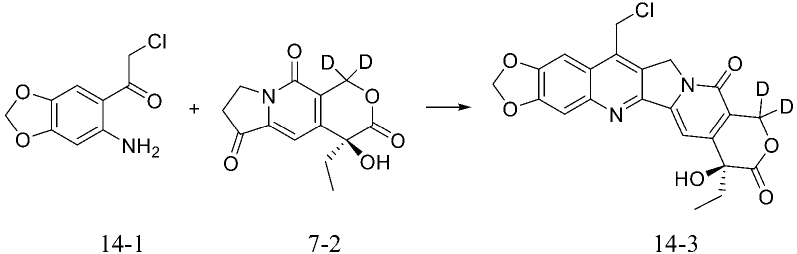

213 mg of compound 14-1, 300 mg of compound 7-2, 7.5 mL of acetic acid, 7.5 mL of toluene, and 68 mg of pyridinium *p*-toluenesulfonate were added to a 50 mL single-necked flask. The reaction was carried out at 110°C for 18 hours. After completion of the reaction (monitored by TLC), the solvent was removed by rotary evaporation, and 300 mg of compound 14-3 was obtained by silica gel column chromatography purification (DCM: MeOH), with a yield of 68%. MS (ESI) m/z [M+H]⁺: 443.1.

### Step 2:

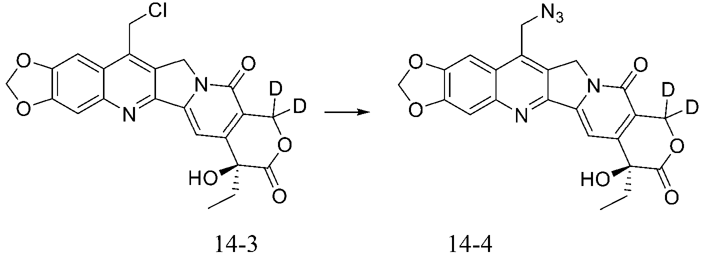

300 mg of compound 14-3 was dissolved in 3 mL of *N,N*-dimethylformamide, followed by the addition of sodium azide (48 mg, 1.1 eq). The reaction was carried out at 80°C for 16 hours. After completion of the reaction (monitored by TLC), the reaction mixture was cooled to room temperature, and 18 mL of water was added. The mixture was extracted with ethyl acetate, and the organic phases were combined. The organic phase was washed with water, dried over anhydrous sodium sulfate, filtered, and rotary evaporated to dryness to obtain 186 mg of crude compound 14-4, with a yield of 63%. MS (ESI) m/z [M+H]⁺: 436.1.

### Step 3:

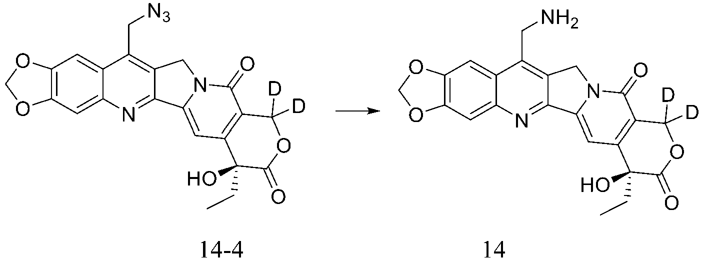

186 mg of compound 14-4 was dissolved in 4 mL of tetrahydrofuran, followed by the addition of triphenylphosphine (134 mg, 1.2 eq). The reaction mixture was stirred at room temperature for 4 hours. After completion of the reaction (monitored by TLC), hydrochloric acid (4 M, 1 mL) was added to the reaction system, and the mixture was reacted at 55°C for 16 hours. After concentration under reduced pressure, the residue was purified by a reverse-phase column (SEPA FLASH SW025, Spherical C18, 20-45 µm, 100 Å; mobile phase A: 0.05% formic acid/water, mobile phase B: acetonitrile; mobile phase A: mobile phase B = 40:60, flow rate 20 mL/min) to obtain 63 mg of compound 14, with a yield of 35%.

¹H NMR (400 MHz, DMSO-*d*₆) δ 8.62 (s, 2H), 8.45 (s, 1H), 7.50 (s, 1H), 7.25 (s, 1H), 6.48 (s, 1H), 6.28 (s, 2H), 5.41 (s, 2H), 5.20 (s, 2H), 4.70 (d, J = 6.0 Hz, 2H), 1.86 (tt, J = 14.2, 6.1 Hz, 2H), 0.88 (t, J = 7.2 Hz, 3H). MS (ESI) m/z [M+H]⁺: 424.1.

### Example 15: Preparation of Compound 15

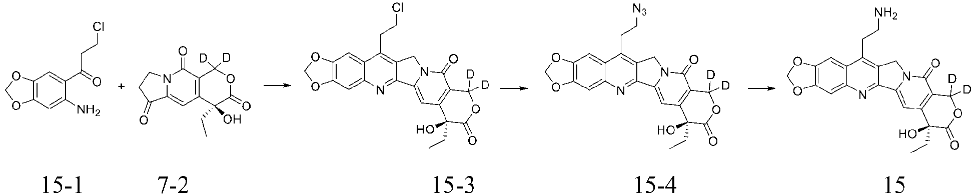

Referring to the preparation method of Example 14, 50 mg of compound 15 was prepared using compounds 15-1 and 7-2 as the starting materials, with a three-step yield of 11%.

¹H NMR (400 MHz, DMSO-*d*₆) δ 8.62 (s, 2H), 8.45 (s, 1H), 7.50 (s, 1H), 7.25 (s, 1H), 6.48 (s, 1H), 6.28 (s, 2H), 5.41 (s, 2H), 5.20 (s, 2H), 3.51-3.46 (m, 2H), 3.17-3.13 (m, 2H), 1.86 (tt, J = 14.2, 6.1 Hz, 2H), 0.88 (t, J = 7.2 Hz, 3H). MS (ESI) m/z [M+H]⁺: 438.2.

### Example 16: Preparation of Compound 16

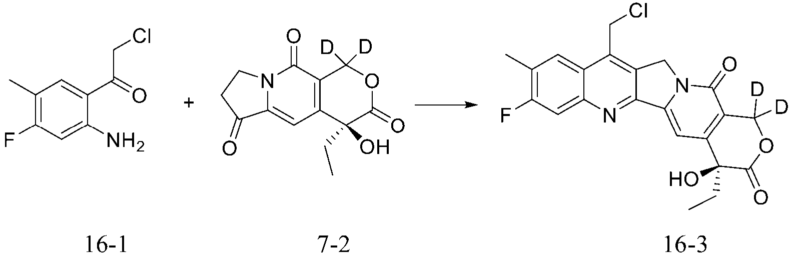

1 g of compound 16-1, 1.5 g of compound 7-2, 30 mL of acetic acid, 30 mL of toluene, and 340 mg of pyridinium p-toluenesulfonate were added to a 250 mL single-necked flask. The reaction was carried out at 110°C for 18 hours. After completion of the reaction (monitored by TLC), the solvent was removed by rotary evaporation, and the target intermediate compound 16-3 (1.6 g) was obtained by silica gel column chromatography purification (dichloromethane: methanol), with a yield of 75%. MS (ESI) m/z [M+H]⁺: 431.1.

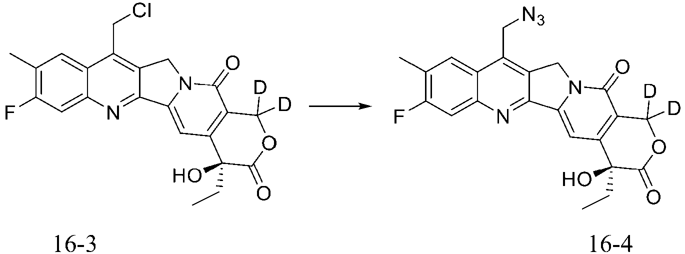

1.6 g of compound 16-3 was dissolved in 16 mL of *N,N-*dimethylformamide, followed by the addition of sodium azide (256 mg, 1.1 eq). The reaction was carried out at 80°C for 16 hours. After completion of the reaction (monitored by TLC), the reaction mixture was cooled to room temperature, and 96 mL of water was added. The mixture was extracted with ethyl acetate, and the organic phases were combined. The organic phase was washed with water, dried over anhydrous sodium sulfate, filtered, and concentrated under reduced pressure to obtain 1.1 g of crude compound 16-4, with a yield of 68%. MS (ESI) m/z [M+H]⁺: 438.2.

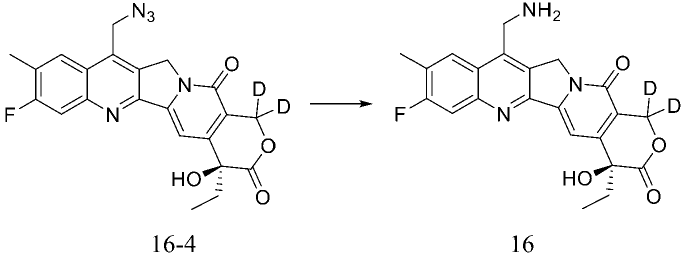

1.1 g of compound 16-4 was dissolved in 22 mL of tetrahydrofuran, followed by the addition of triphenylphosphine (0.79 g, 1.2 eq). The mixture was stirred at room temperature for 4 hours. After completion of the reaction (monitored by TLC), hydrochloric acid (4 M, 5 mL) was added to the reaction system, and the reaction was carried out at 55°C for 16 hours. The reaction mixture was concentrated under reduced pressure and purified by a reverse-phase column (instrument model: SEPA FLASH SW025, chromatographic column: Spherical C18, 20-45 µm, 100 Å, mobile phase A: 0.05% formic acid/water, mobile phase B: acetonitrile; mobile phase A: mobile phase B = 45:55, flow rate: 20 mL/min) to obtain 0.58 g of compound 16, with a yield of 56%.

¹H NMR (400 MHz, DMSO-*d*₆) δ 8.62 (s, 3H), 8.43 (d, J = 8.0 Hz, 1H), 7.99 (d, J = 10.7 Hz, 1H), 7.35 (s, 1H), 5.59 (s, 2H), 4.70 (d, J = 6.0 Hz, 2H), 2.55 (s, 3H), 1.88 (hept, J = 7.1 Hz, 2H), 0.88 (t, J = 7.3 Hz, 3H). MS (ESI) m/z [M+H]⁺: 412.2.

### Example 17: Preparation of Compound 17

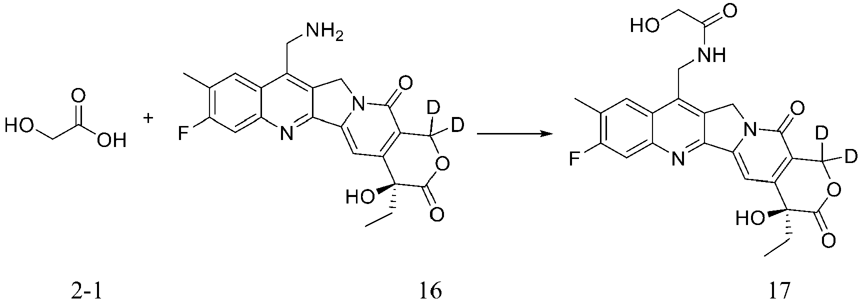

2.4 mL of water and 3.0 mL of tetrahydrofuran were added to a mixture of anhydrous sodium sulfate (2.25 eq), 2-hydroxyacetic acid (1.45 eq), and compound 16 (100 mg). After stirring at room temperature for 15 minutes, N-methylmorpholine (1.10 eq) was added, and the mixture was stirred at room temperature for 15 minutes. Subsequently, EDCI (2 eq) was added, and the reaction mixture was stirred at room temperature for 3 hours. After the complete reaction of compound 16 was monitored by HPLC, the mixture was extracted with dichloromethane, dried over anhydrous sodium sulfate, and filtered. The solvent was removed by rotary evaporation, and 82 mg of compound 17 was obtained by silica gel column chromatography purification (DCM: MeOH), with a yield of 72%.

¹H NMR (400 MHz, DMSO) δ 8.41 (d, J = 8.9 Hz, 1H), 7.77 (d, J = 11.0 Hz, 1H), 7.30 (s, 1H), 6.52 (s, 1H), 5.58 (d, J = 5.4 Hz, 1H), 5.49 (t, J = 5.7 Hz, 1H), 5.20 (t, J = 13.9 Hz, 2H), 3.96 (d, J = 5.5 Hz, 2H), 2.55 (s, 3H), 1.96 - 1.78 (m, 2H), 0.87 (t, J = 7.2 Hz, 3H). MS (ESI) m/z [M+H]⁺: 470.2.

### Example 18: Preparation of Compound 18

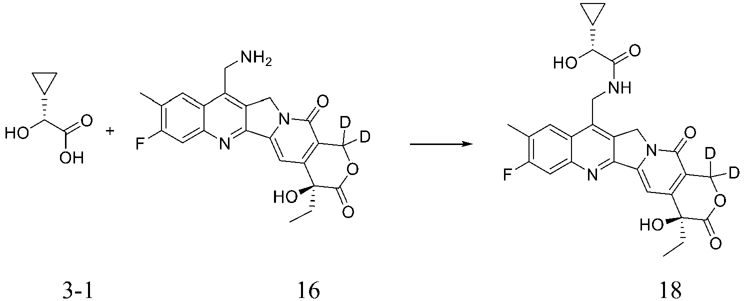

Referring to the preparation method of Example 17, 78 mg of compound 18 was prepared using compound 3-1 and compound 16 as the starting materials, with a yield of 63%.

¹H NMR (400 MHz, DMSO-*d*₆) δ 8.36 (d, J = 8.4 Hz, 1H), 7.78 (d, J = 10.9 Hz, 1H), 7.31 (s, 1H), 6.52 (s, 0H), 5.58 - 5.48 (m, 1H), 5.38 (d, J = 5.3 Hz, 1H), 5.21 (q, J = 19.0 Hz, 2H), 3.62 (d, J = 5.2 Hz, 1H), 2.39 (s, 4H), 1.86 (dt, J = 14.3, 7.1 Hz, 2H), 1.14 (q, J = 7.3 Hz, 2H), 0.87 (t, J = 7.2 Hz, 3H), 0.38 (dd, J = 20.9, 5.6 Hz, 4H). MS (ESI) m/z [M+H]⁺: 510.2.

### Example 19: Preparation of Compound 19

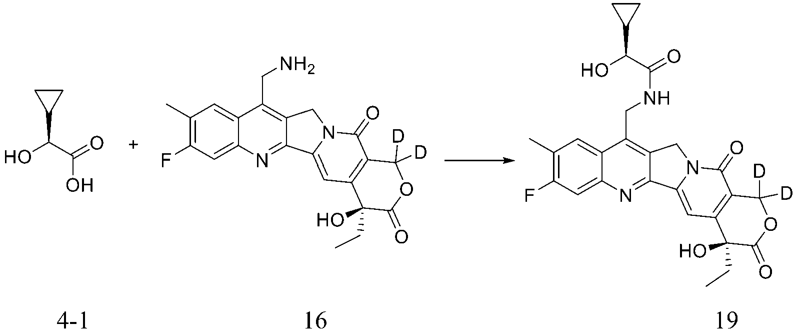

Referring to the preparation method of Example 17, 74 mg of compound 19 was prepared using compound 4-1 and compound 16 as the starting materials, with a yield of 60%.

¹H NMR (400 MHz, DMSO-*d*₆) δ 8.37 (d, J = 9.0 Hz, 1H), 7.77 (d, J = 11.0 Hz, 1H), 7.30 (s, 1H), 6.51 (s, 1H), 5.58 (q, J = 6.7 Hz, 1H), 5.49 (d, J = 5.2 Hz, 1H), 5.35 - 5.06 (m, 2H), 3.60 (t, J = 5.7 Hz, 1H), 2.39 (s, 3H), 1.85 (dq, J = 14.2, 7.1 Hz, 2H), 1.24 (q, J = 8.1 Hz, 1H), 0.87 (t, J = 7.3 Hz, 3H), 0.59 - 0.32 (m, 4H). MS (ESI) m/z [M+H]⁺: 510.2.

### Example 20: Preparation of Compound 20

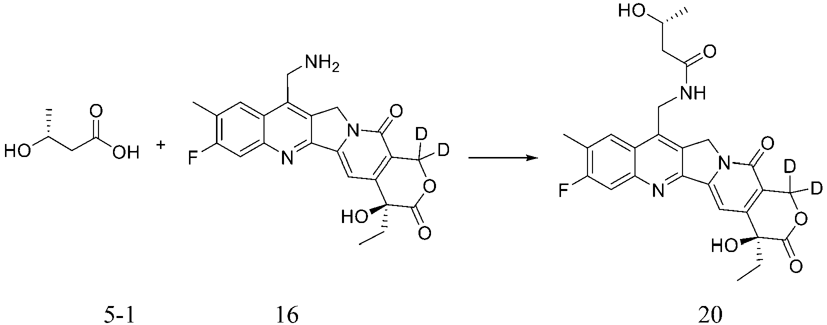

Referring to the preparation method of Example 17, 70 mg of compound 20 was prepared using compound 5-1 and compound 16 as the starting materials, with a yield of 58%.

¹H NMR (400 MHz, DMSO-*d*₆) δ 8.42 (d, J = 8.7 Hz, 1H), 7.78 (d, J = 11.0 Hz, 1H), 7.30 (s, 1H), 6.52 (s, 1H), 5.55 (dt, J = 9.3, 5.0 Hz, 1H), 5.29 - 5.12 (m, 2H), 4.66 (d, J = 4.7 Hz, 1H), 4.05 (dt, J = 12.1, 6.0 Hz, 1H), 2.39 (s, 3H), 2.13 (dd, J = 10.7, 5.4 Hz, 2H), 1.86 (hept, J = 7.1 Hz, 2H), 1.09 (d, J = 6.2 Hz, 3H), 0.87 (t, J = 7.3 Hz, 3H). MS (ESI) m/z [M+H]⁺: 498.2.

### Example 21: Preparation of Compound 21

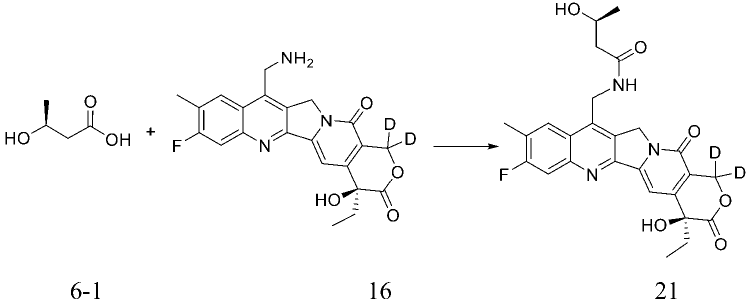

Referring to the preparation method of Example 17, 75 mg of compound 21 was prepared using compound 6-1 and compound 16 as the starting materials, with a yield of 62%.

¹H NMR (400 MHz, DMSO-*d*₆) δ 8.41 (d, J = 8.7 Hz, 1H), 7.78 (d, J = 11.0 Hz, 1H), 7.30 (s, 1H), 6.53 (s, 1H), 5.57 (dt, J = 8.8, 4.5 Hz, 1H), 5.22 (d, J = 2.7 Hz, 2H), 4.64 (d, J = 4.5 Hz, 1H), 4.05 (dt, J = 11.9, 6.1 Hz, 1H), 2.39 (s, 3H), 2.17 - 2.04 (m, 2H), 1.86 (hept, J = 7.1 Hz, 2H), 1.08 (d, J = 6.1 Hz, 3H), 0.87 (t, J = 7.3 Hz, 3H). MS (ESI) m/z [M+H]⁺: 498.2.

### Biological activity test

### 1. In vitro antitumor activity detection of the payload compounds of the present disclosure

Objective: To detect the *in vitro* inhibitory activity of the compounds of the present disclosure against NCI-N87 (human gastric cancer cells), Calu-3 (human lung adenocarcinoma cells), MDA-MB-453 (human breast cancer cells), KPL-4 (human breast cancer cells), or MDA-MB-468 (human breast cancer cells).

Tumor cells in the logarithmic growth phase, including NCI-N87 (source: ATCC, catalog number: CRL-5822), Calu-3 (source: ATCC, catalog number: HTB-55), MDA-MB-453 (source: ATCC, catalog number: HTB-131), KPL-4 (source: Nanjing Cobioer Biosciences, catalog number: CBP60379), or MDA-MB-468 (source: ATCC, catalog number: HTB-132), were seeded into cell plates at a density of 5,000 cells/well. The cell plates were then incubated in a 37°C, 5% CO₂ cell culture incubator for 12-16 hours. 100 µL of sample (starting at 10 µM, 4-fold dilution, 10 concentrations) was added to each well. After gentle shaking, the plates were placed in the incubator for further incubation. After 48 hours of incubation, 70 µL of CellTiter-Glo^{™} (Promega, catalog number: G7572) working solution was added, gently shaken to lyse the cells, and the plates were read on a microplate reader. The cell proliferation inhibition rate was calculated using the following formula: cell proliferation inhibition rate = (1-sample well/control well)×100%. GraphPad Prism 8.0 software was used to plot the Log value of sample concentration as the x-axis and Cytotoxicity% as the y-axis. The data were analyzed by Nonlinear regression (curve fit) to obtain the IC₅₀ values for each sample, with specific results shown in Tables 1-1 and 1-2.

**Table 1-1. In vitro activity test results of the compounds of the present disclosure**

| **Compound** | **NCI-N87** | **Calu-3** | **MDA-MB-453** | **MDA-MB-468** |
|---|---|---|---|---|
| | **IC₅₀ (nM)** | **IC₅₀ (nM)** | **IC₅₀ (nM)** | **IC₅₀ (nM)** |
| 2 | 7.17 | 27.15 | 114.5 | 0.98 |
| 3 | 4.37 | 17.13 | 40.27 | 0.97 |
| 4 | 4.06 | 19.32 | 55.34 | 0.88 |
| 5 | 9.18 | 32.9 | 94.6 | 1.3 |
| 6 | 13.35 | 46.63 | 121.1 | 3.11 |

**Table 1-2. In vitro activity test results of the compounds of the present disclosure**

| **Compound** | NCI-N87 | KPL-4 | MDA-MB-468 |
|---|---|---|---|
| | IC₅₀ (nM) | IC₅₀ (nM) | IC₅₀ (nM) |
| 12 | 7.29 | 7.05 | 1.7 |

The experimental results show that the compounds of the present disclosure exhibit significant proliferation inhibitory activity against NCI-N87, Calu-3, MDA-MB-453, KPL-4, or MDA-MB-468.

### 2. Pharmacokinetics of the compounds of the present disclosure in mice

Objective: To evaluate the pharmacokinetic characteristics of the compounds of the present disclosure in mice.

Balb/c mice were taken, with 12 mice per group (half male and half female), randomly grouped, and administered a single injection of the sample (Formulation A: 5% DMSO + 95% (15% sulfobutyl-β-cyclodextrin-normal saline), Formulation B: 5% DMSO-10% Solutol HS 15-85% normal saline, diluted according to the dose). Blood samples were collected at crossover time points, including before administration and at 5 min, 30 min, 1 h, 2 h, 4 h, 6 h, 8 h, 10 h, and 24 h after administration. Plasma was collected, and the sample concentration in plasma was detected by LC-MS. The pharmacokinetic parameters were calculated, and the results are shown in Table 2-1.

**Table 2-1. PK parameters of the compounds of the present disclosure in mice**

| **Compound** | **Mouse PK (IV, 3 mpk)** | | |
|---|---|---|---|
| | **T_{1/2} (h)** | **Cₘₐₓ (ng/mL)** | **Formulation** |
| Dxd | 1.17 | 521 | A |
| 2 | 0.455 | 449 | A |
| 3 | 0.607 | 4305 | B |
| 5 | 0.674 | 3515 | B |
| 6 | 0.679 | 2582.5 | B |

The experimental results show that the compounds of the present disclosure exhibit a short *in vivo* half-life in mice. When these compounds are used as payloads for ADCs, in the event of payload release from the ADC *in vivo,* the payload is capable of being rapidly eliminated *in vivo,* thereby exhibiting superior *in vivo* safety.

### 3. Pharmacokinetics of the compounds of the present disclosure in rats

Objective: To evaluate the pharmacokinetic characteristics of the compounds of the present disclosure in rats.

SD rats (purchased from Beijing Vital River Laboratory Animal Technology Co., Ltd.) were used, with 6 rats per group (half male and half female), randomly grouped. A single injection of the sample (Formulation A: 5% DMSO + 95% (15% sulfobutyl-β-cyclodextrin-normal saline), diluted according to the dose) was administered. Blood samples were collected at crossover time points: before administration and at 5 min, 30 min, 1 h, 2 h, 4 h, 6 h, 8 h, 10 h, and 24 h after administration. Plasma was collected, and the sample concentration in plasma was detected by LC-MS. Pharmacokinetic parameters were calculated, and the results are shown in Table 3-1.

**Table 3-1. PK parameters of the compounds of the present disclosure in rats**

| **Compound** | **Rat PK (IV, 3 mpk)** | | | | |
|---|---|---|---|---|---|
| | **T_{1/2} (h)** | **Cmax (ng/mL)** | **AUCinf (h*ng/mL)** | **Cl (mL/h/kg)** | **Formulation** |
| Dxd | 1.172 | 621.2 | 352.1 | 8629.5 | A |
| 2 | 0.661 | 406.2 | 217.7 | 14072.1 | A |

The experimental results show that the compounds of the present disclosure also exhibit a short *in vivo* half-life and a rapid *in vivo* clearance rate in rats.

### 4. In vitro plasma stability of the compounds of the present disclosure

398 µL of plasma was taken and preheated to 37°C, then co-incubated for 15 minutes. 2 µL of the sample solution was added to the plasma to prepare a final sample concentration of 5 µM. The above solution was co-incubated at 37°C, and 50 µL aliquots were taken at 0, 15, 30, 60, and 120 minutes, followed by the addition of 450 µL of ice-cold acetonitrile (containing the internal standard). The solution was vortexed for 10 minutes, centrifuged, and the remaining content of the parent substrate was detected by the LC-MS/MS method. For specific results, refer to Table 4-1.

**Table 4-1. Plasma stability of the compounds of the present disclosure**

| Compound | Residual content of parent toxin at 120 minutes (relative to 0 minutes) | |
|---|---|---|
| | Human plasma | CD1 mouse plasma |
| Dxd | 87.57% | 95.42% |
| 2 | 111.04% | 102.54% |
| 3 | 105.54% | 104.97% |
| 5 | 107.73% | 96.02% |
| 6 | 94.89% | 105.93% |

The experimental results show that the compounds of the present disclosure exhibit good plasma stability.

### 5. Repeated administration toxicity experiments of the compounds of the present disclosure in rats

Test sample preparation: The test sample was weighed according to the predetermined weight into a glass vial. First, 1 mL of DMSO was added and mixed until the test sample was dissolved, followed by the addition of 1 mL of Solutol HS-15. After mixing thoroughly, 8 mL of sodium chloride injection was added and vortex-mixed to obtain a clear and transparent solution.

SD rats (purchased from Beijing Vital River) were used as test animals. Compound 2 and the positive control Dxd were administered via tail vein injection for 7 consecutive days, with both compound 2 and the positive control Dxd set at two doses of 0.3 mg/kg and 1.0 mg/kg. During the acclimation period, one cage-side observation was conducted daily; during the administration period, all animals were subjected to clinical observations twice daily (morning and afternoon). Reactions in various aspects, including the skin, fur, eyes (sclera), ears, nose, oral cavity, chest, abdomen, urogenital area, limbs, as well as respiration, movement, urination, defecation (including urine and feces color), and behavioral changes, were recorded. Daily weight data of the main test group animals were documented. For animals scheduled for dissection, terminal body weight (fasted) was measured before dissection. The food intake of the test animals was also recorded. The specific experimental results are shown in Table 5-1.

**Table 5-1. Results of repeated administration toxicity experiments of the compounds of the present disclosure in rats**

| Observation indicators | Test results | |
|---|---|---|
| | Control Dxd group | Compound 2 group |
| Clinical observation | Main trial group + TK group: | Main trial group + TK group: |
| | Dxd: 0.3 mpk dose group: 31% (5/16) animals showed ataxia immediately after administration, among which 40% (2/5) died during recovery, and the other rats gradually recovered; | Compound 2: 0.3 mpk dose group: 13% (2/16) animals showed ataxia immediately after administration, then gradually recovered; |
| | | Compound 2: 1 mpk dose group: 25% (4/16) animals showed ataxia immediately after administration, then gradually recovered; |
| | Dxd: 1 mpk dose group: 31% (5/16) animals showed ataxia immediately after administration, among which 40% (2/5) died during recovery, and the other rats gradually recovered; | |
| Body weight, food intake | Slow weight gain/weight loss; | At the same dose, the effects of Compound 2 and Dxd on rat body weight and food intake were generally comparable; |
| | Decreased food intake | |
| | | For ♀ rats, the average food intake in the Compound 2 group was higher than that in the control group at the same dose; |

wherein ♂ represents male, and ♀ represents female.

At the end of the administration, gross dissection was performed on D8 after administration. Focal/punctate dark red discoloration was observed in the lungs of some deceased rats in the Dxd control group, while no such pathological changes were found in any dose group of compound 2.

The above experimental data show that compound 2 of the present disclosure exhibits lower *in vivo* toxicity and superior *in vivo* safety compared to the positive control Dxd.

In the present disclosure, the comparative compound Dxd refers to the following compound, which can be prepared according to the published literature WO2014057687A.

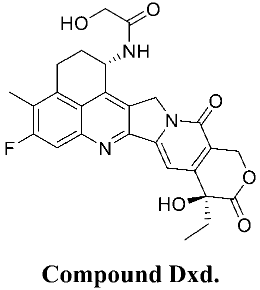

## Claims

1. A compound of Formula II, a pharmaceutically acceptable salt thereof, or a stereoisomer thereof: 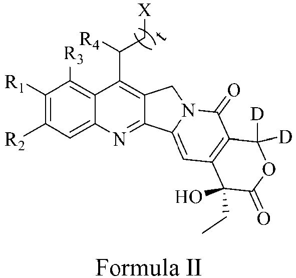
wherein R₁ is H, halogen, OH, SH, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, C₁₋₄ alkoxy, C₁₋₄ haloalkyl, or C₁₋₄ haloalkoxy;
or R₃ and R₄ are cyclized to form -(CH₂)ₖ-, wherein k is 1, 2, 3, or 4;
X is H, OH, HO-CH(R₅)-(CH₂)ₚ-CO-NH-, or -N(R₆)(R₇), wherein p is 0, 1, or 2;
R₅ is H, C₁₋₄ alkyl, C₁₋₄ haloalkyl, 3- to 6-membered cycloalkyl, or 3- to 6-membered heterocycloalkyl;
R₆ is H, C₁₋₄ alkyl, or C₁₋₄ haloalkyl;
R₇ is H or R₈-S(O)₂-;
R₈ is C₁₋₄ alkyl; and
t is 0, 1, 2, 3, 4, or 5;
and the compound of Formula II excludes any one of the following compounds:

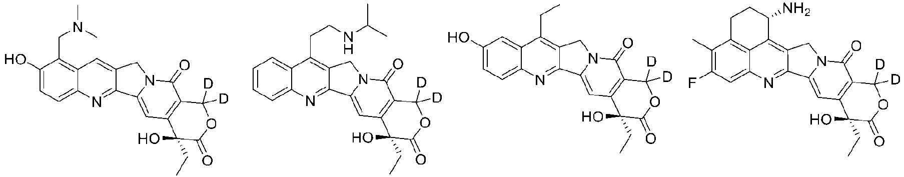

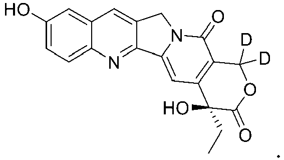

2. The compound, the pharmaceutically acceptable salt thereof, or the stereoisomer thereof according to claim 1, wherein R₁ is C₁₋₄ alkyl, and R₂ is halogen; or R₁ is methyl, and R₂ is F; or R₁ is H, and R₂ is H; or R₁ and R₂ are cyclized to form -O-CH₂-O-; or R₁ is NH₂, and R₂ is H or halogen; or R₁ is NH₂, and R₂ is H or F.

3. The compound, the pharmaceutically acceptable salt thereof, or the stereoisomer thereof according to claim 1 or 2, wherein R₃ is H, and R₄ is H; or R₃ is H, and R₄ is C₁₋₄ alkyl; or R₃ is H, and R₄ is methyl; or R₃ and R₄ are cyclized to form -CH₂-CH₂-.

4. The compound, the pharmaceutically acceptable salt thereof, or the stereoisomer thereof according to any one of claims 1 to 3, wherein X is HO-CH(R₅)-(CH₂)ₚ-CO-NH-, and p is 0, 1, or 2; or X is OH or NH₂; or X is H, and t is 0; or X is -N(R₆)(R₇).

5. The compound, the pharmaceutically acceptable salt thereof, or the stereoisomer thereof according to any one of claims 1 to 3, wherein the compound of Formula II is a compound of Formula IIa, a pharmaceutically acceptable salt thereof, or a stereoisomer thereof: 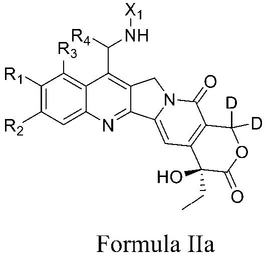 wherein R₁, R₂, R₃, and R₄ are as defined in any one of claims 1 to 3, and X₁ is H, HO-CH(R₅)-(CH₂)ₚ-CO-, wherein p is 0, 1, or 2; R₅ is H, C₁₋₄ alkyl, 3- to 6-membered cycloalkyl, or 3- to 6-membered heterocycloalkyl.

6. The compound, the pharmaceutically acceptable salt thereof, or the stereoisomer thereof according to claim 5, wherein R₁ is methyl or methoxy; or R₁ is methyl.

7. The compound, the pharmaceutically acceptable salt thereof, or the stereoisomer thereof according to claim 5 or 6, wherein R₂ is F or Cl; or R₂ is F.

8. The compound, the pharmaceutically acceptable salt thereof, or the stereoisomer thereof according to claim 5, wherein R₁ is methyl, and R₂ is F.

9. The compound, the pharmaceutically acceptable salt thereof, or the stereoisomer thereof according to any one of claims 5 to 8, wherein R₃ and R₄ are cyclized to form -(CH₂)ₖ-, and k is 2; or R₃ is H, and R₄ is H.

10. The compound, the pharmaceutically acceptable salt thereof, or the stereoisomer thereof according to any one of claims 5 to 9, wherein X₁ is HO-CH(R₅)-(CH₂)ₚ-CO-, p is 0 or 1, and R₅ is H, C₁₋₄ alkyl, or 3- to 6-membered cycloalkyl; or X₁ is HO-CH(R₅)-(CH₂)ₚ-CO-, p is 0 or 1, and R₅ is H, methyl, or cyclopropyl; or X₁ is HO-CH(R₅)-CO-, and R₅ is H or 3- to 6-membered cycloalkyl; or X₁ is HO-CH(R₅)-CO-, and R₅ is H or cyclopropyl; or X₁ is HO-CH(R₅)-CH₂-CO-, and R₅ is C₁₋₄ alkyl; or X₁ is HO-CH(R₅)-CH₂-CO-, and R₅ is methyl; or X₁ is the following group: H,

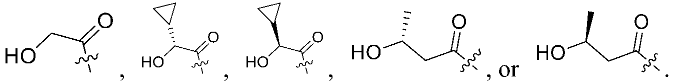

11. The compound, the pharmaceutically acceptable salt thereof, or the stereoisomer thereof according to any one of claims 5 to 10, wherein the compound of Formula IIa is a compound of Formula IIa-1 or Formula IIa-2, a pharmaceutically acceptable salt thereof, or a stereoisomer thereof; 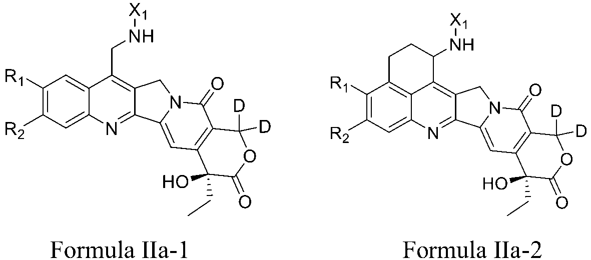 wherein R₁, R₂, and X₁ are as defined in any one of claims 5 to 8 and 10.

12. The compound, the pharmaceutically acceptable salt thereof, or the stereoisomer thereof according to any one of claims 1 to 3, wherein the compound of Formula II is a compound of Formula IIb, a pharmaceutically acceptable salt thereof, or a stereoisomer thereof: 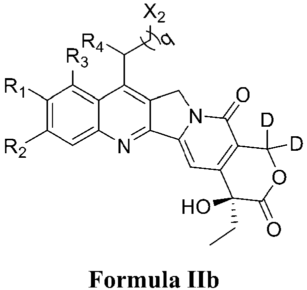 wherein R₁, R₂, R₃, and R₄ are as defined in any one of claims 1 to 3; X₂ is H, OH, or - N(R₆)(R₇); R₆ is H or C₁₋₄ alkyl; R₇ is H or R₈-S(O)₂-; R₈ is C₁₋₄ alkyl; and q is 0, 1, 2, 3, or 4.

13. The compound, the pharmaceutically acceptable salt thereof, or the stereoisomer thereof according to claim 12, wherein R₁ is methyl, and R₂ is H, Cl, or F; or R₁ and R₂ are cyclized to form -O-(CH₂)ₘ-O-, and m is 1 or 2.

14. The compound, the pharmaceutically acceptable salt thereof, or the stereoisomer thereof according to claim 12 or 13, wherein R₃ and R₄ are both H; or R₃ is H, and R₄ is methyl.

15. The compound, the pharmaceutically acceptable salt thereof, or the stereoisomer thereof according to any one of claims 12 to 14, wherein X₂ is H, and q is 0; or X₂ is OH or - N(R₆)(R₇), and q is 0, 1, 2, 3, or 4; or X₂ is OH or -N(R₆)(R₇), q is 0, 1, 2, 3, or 4, R₃ and R₄ are both H, R₁ is methyl and R₂ is H, Cl, or F, or R₁ and R₂ are cyclized to form -O-(CH₂)ₘ-O-, wherein m is 1 or 2; or X₂ is H, OH, NH₂, or N(i-Pr)-S(O)₂-CH₃.

16. The compound, the pharmaceutically acceptable salt thereof, or the stereoisomer thereof according to any one of claims 12 to 15, wherein the Formula IIb is a compound of Formula IIb-1 or Formula IIb-2, a pharmaceutically acceptable salt thereof, or a stereoisomer thereof; 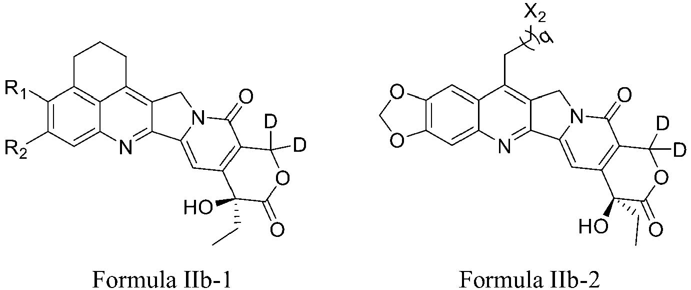 wherein R₁, R₂, X₂, and q are as defined in any one of claims 12 to 13 and 15.

17. The compound, the pharmaceutically acceptable salt thereof, or the stereoisomer thereof according to claim 5 or 12, wherein the compound of Formula IIa is the following compound, a pharmaceutically acceptable salt thereof, or a stereoisomer thereof: 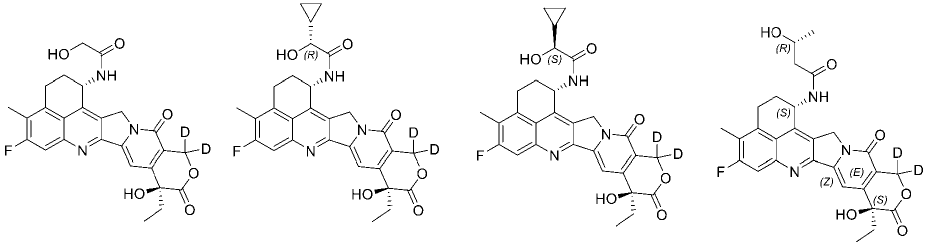 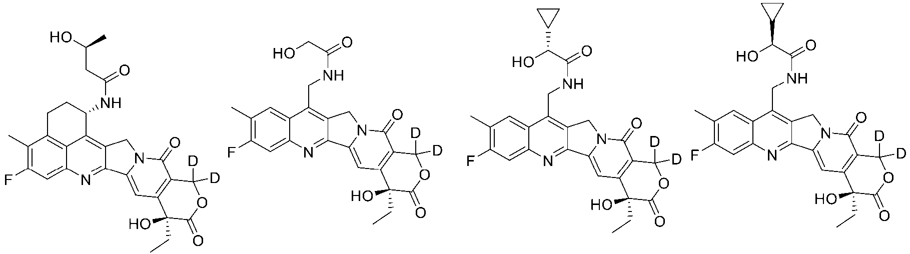 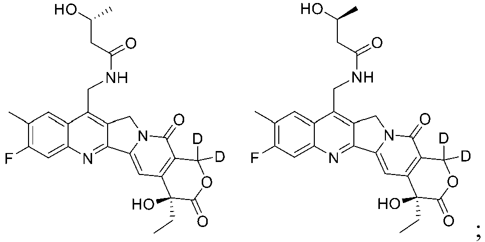 the Formula IIb is the following compound, a pharmaceutically acceptable salt thereof, or a stereoisomer thereof:

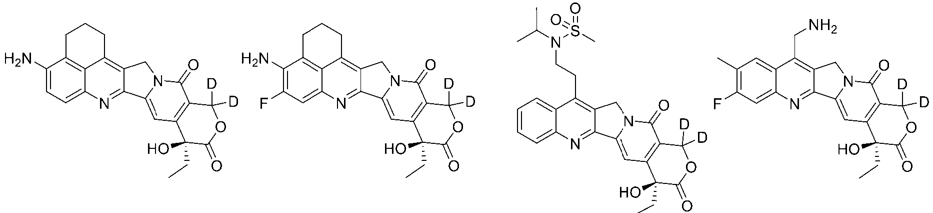

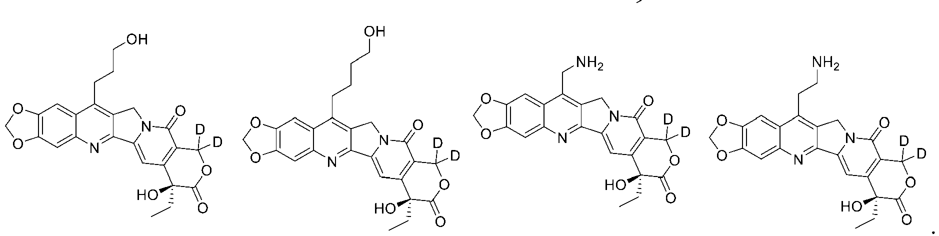

18. A pharmaceutical composition comprising the compound, the pharmaceutically acceptable salt thereof, or the stereoisomer thereof as defined in any one of claims 1 to 17, and a pharmaceutically acceptable excipient.

19. Use of the compound, the pharmaceutically acceptable salt thereof, or the stereoisomer thereof as defined in any one of claims 1 to 17, or the pharmaceutical composition as defined in claim 18, in the manufacture of a medicament for treating tumors.

20. A method for treating tumors, comprising administering to an individual in need thereof the compound, the pharmaceutically acceptable salt thereof, or the stereoisomer thereof as defined in any one of claims 1 to 17, or the pharmaceutical composition as defined in claim 18.
